# EUROPEAN PATENT APPLICATION

(11) **EP 4 652 953 A1**
(43) Date of publication of application: **26.11.2025**
(21) Application number: 25167576.5
(22) Date of filing: 31.03.2025
(51) Int. Cl.: A61B 34/10, A61B 34/00, A61B 17/16

(54) **DEVICES, SYSTEMS, AND METHODS FOR BONE BALANCE ADJUSTMENT BASED ON LIGAMENT LAXITY**

(30) Priority: 29.03.2024 US 202463571895 P
(71) Applicant: Mako Surgical Corporation, Weston, Florida 33331 (US)
(72) Inventor: BONO, James, Dover, MA 02030 (US); BONO, Olivia Jane, Dover, MA 02030 (US)
(74) Representative: Purdylucey Intellectual Property

(57) **Abstract**

A method of assessing a joint may include identifying a first bone portion in an image of the joint. The method may further include identifying a cross-sectional area of the first bone portion and identifying a medial ligament in extension value, a lateral ligament in extension value, a medial ligament in flexion value, and a lateral side in flexion value; determining a lateral ligament laxity based on the lateral ligament in extension value; and determining one or more adjustment parameters based on the identified cross-sectional area and the lateral ligament laxity. The determining the adjustment parameters may include a predicted change in soft tissue laxity after the identified first bone portion is removed. The adjustment parameters may include an adjustment to a planned bone resection and/or an adjustment to a planned thickness of an implant. The method may further include outputting the determined adjustment parameters to a display.

## Description

### FIELD OF THE DISCLOSURE

The present disclosure relates to systems, devices, and methods for optimizing medical procedures, and in particular to systems, devices, and methods for planning, guiding, and/or facilitating procedures on a joint, such as a knee, to optimize outcomes, among other aspects.

### BACKGROUND OF THE DISCLOSURE

Surgeries incorporating prosthetics and/or implants, such as joint replacement procedures, often require careful consideration of various factors. For example, when cutting or resecting a bone for placement of a prosthesis, ligament laxity and/or joint subluxation may need to be calculated and/or adjusted to properly balance a joint (e.g., a knee joint). Improved systems and methods for performing procedures on a joint, collecting patient data, and analyzing image data and/or ligament laxity data and/or joint subluxation data and/or bone resection data are desired to help in planning and conducting surgeries, to improve patient outcomes, to decrease procedure time, to reduce post-surgery complications, among other benefits.

### BRIEF SUMMARY OF THE DISCLOSURE

In an aspect of the present disclosure, a method of assessing a joint may include identifying a first bone portion in an image of the joint. The first bone portion may be positioned under a soft tissue. The method may further include identifying a cross-sectional area of the first bone portion and identifying a medial ligament in extension value, a lateral ligament in extension value, and a medial ligament in flexion value, a lateral side in flexion value associated with the joint, determining a lateral ligament laxity based on the lateral ligament in extension value, determining one or more adjustment parameters based on the identified cross-sectional area of the first bone portion and the lateral ligament laxity. The determining the one or more adjustment parameters may include applying a linear equation that receives, as input, the identified cross-sectional area and the lateral ligament laxity, and the one or more adjustment parameters may include a predicted change in soft tissue laxity after the identified first bone portion is removed. The soft tissue laxity may include the lateral ligament laxity. The one or more adjustment parameters may further include an adjustment to a planned bone resection of one or more bone cuts, an adjustment to a planned bone resection angle of the one or more bone cuts, and/or an adjustment to a planned thickness of an implant. The method may further include outputting the one or more determined adjustment parameters to a display.

Identifying the first bone portion and/or identifying the cross-sectional area of the first bone portion may include analyzing the image using one or more image processing techniques.

Identifying the cross-sectional area of the first bone portion may include analyzing a first dimension of the first bone portion and a second dimension of the first bone portion, and disregarding a third dimension of the first bone portion.

The one or more adjustment parameters may include an adjustment to a planned bone resection depth of the one or more bone cuts and an adjustment to a planned bone resection angle of the one or more bone cuts.

The method may further include identifying a ligament in the image of the joint, identifying a measurement of the ligament and an amount of subluxation of the first bone portion, and identifying an amount of attenuation of the ligament, based on the measurement of the ligament and the amount of subluxation of the first bone portion. The determining one or more adjustment parameters may be further based on the amount of attenuation of the joint. The linear equation may further receive as input the amount of attenuation of the joint.

The linear equation may include a linear relationship between the identified cross-sectional area and the adjustment to the planned bone resection depth such that, the greater the identified cross-sectional area, the greater the decrease in planned bone resection depth, and/or a linear relationship between the identified cross-sectional area and the planned thickness of the implant such that, the greater the identified cross-sectional area, the greater the increase to the planned thickness of the implant.

The method may further include identifying a second bone portion in the image of the joint that is positioned under the soft tissue, identifying a cross-sectional area of the second bone portion, identifying a position of the first bone portion, and identifying a position of the second bone portion. Executing the second algorithm to determine the one or more adjustment parameters may be further based on the identified cross-sectional area of the second bone portion, the identified position of the first bone portion, and the identified position of the second bone portion.

The method may further include determining, based on the identified position of the first bone portion and the identified position of the second bone portion, the first bone portion is provided at a first side of the joint and the second bone portion is provided at a second side of the joint opposite the first side, and determining a difference in the identified cross-sectional area of the first bone portion and the identified cross-sectional area of the second bone portion. The linear equation may include a linear relationship between the determined difference in the identified cross-sectional areas and the adjustment to the planned bone resection angle such that, the greater the determined difference in the identified cross-sectional areas, the greater the adjustment to the planned bone resection angle.

The method may further include determining whether a difference in the identified cross-sectional areas is greater than or equal to a predetermined difference threshold. If the determined difference in the identified cross-sectional area is not greater than or equal to the predetermined difference threshold, determining that the first bone portion and the second bone portion are symmetric, and if the determined difference in the identified cross-sectional area is greater than the predetermined difference threshold, determining that the first bone portion and the second bone portion are asymmetric.

If the first bone portion and the second bone portion are determined to be symmetric, then executing the second algorithm may include determining that the planned bone resection depth should be decreased by a predetermined amount of depth per a predetermined amount of the identified cross-sectional area of the first bone portion and/or the second bone portion, or determining that the planned thickness of the implant should be increased by the predetermined amount of depth per the predetermined amount of the identified cross-sectional area of the first bone portion and/or the second bone portion, and if the first bone portion and the second bone portion are determined to be asymmetric, then executing the second algorithm may include determining, based on the identified cross-sectional area of the first bone portion and the identified cross-sectional area of the second bone portion, whether the first bone portion is larger than the second bone portion, if the first bone portion is determined to be larger than the second bone portion, determining that the planned bone resection angle should be adjusted in a first direction or orientation by a predetermined amount of bone resection angle per a predetermined amount of difference between the identified cross-sectional area of the first bone portion and the identified cross-sectional area of the second bone portion, and if the first bone portion is determined not to be larger than the second bone portion, determining that the second bone portion is larger than the first bone portion, and determining that the planned bone resection angle should be adjusted in a second direction or orientation opposite the first direction or orientation by the predetermined amount of bone resection angle per the predetermined amount of difference.

The method may further include determining that the first bone portion and the second bone portion are asymmetric, determining that both the identified cross-sectional area of the first bone portion and the identified cross-sectional area of the second bone portion are greater than a predetermined cross-sectional area, determining that the difference in the identified cross-sectional areas is greater than a predetermined difference, and determining that the planned bone resection depth should be decreased by the predetermined amount of depth per the predetermined amount of the identified cross-sectional area of the first bone portion and/or the second bone portion, or determining that the planned thickness of the implant should be increased by the predetermined amount of depth per the predetermined amount of the identified cross-sectional area of the first bone portion and/or the second bone portion.

The joint may be a knee joint, and the first bone portion may be a lateral bone portion and the second bone portion may be a medial bone portion, the one or more bone cuts may include a tibial bone cut or a femoral bone cut, the first direction may be a tibial varus or a femoral varus, and the second direction may be a tibial valgus or a femoral valgus.

The predetermined amount of depth may be in a range of 0.4 millimeters (mm) to 0.6 mm, the predetermined amount of the identified cross-sectional area of the first bone portion and/or the second bone portion may be in a range of 85 mm2 to 100 mm2, the predetermined amount of bone resection angle may be in a range of 0.4° to 0.6°, and the predetermined amount of difference between the identified cross-sectional area of the first bone portion and the identified cross-sectional area of the second bone portion may be in a range of 80 mm2 to 100 mm2.

The predetermined amount of depth may be in a range of 0.075 mm - 0.125 mm, the predetermined amount of the identified cross-sectional area of the first bone portion and/or the second bone portion may be in a range of 15 mm2 to 25 mm2, the predetermined amount of bone resection angle may be in a range of 0.075° - 0.125°, and the predetermined amount of difference between the identified cross-sectional area of the first bone portion and the identified cross-sectional area of the second bone portion may be in a range of 15 mm2 to 25 mm2.

According to another aspect of the present disclosure, a method of assessing a joint may include identifying a first bone portion and a ligament in an image of the joint. The first bone portion may be positioned under the ligament. The method may further include identifying a cross-sectional area of the first bone portion and identifying a measurement of the ligament and an amount of subluxation of the first bone portion, determining an amount of attenuation of the ligament based on the measurement of the ligament and the amount of subluxation of the first bone portion, determining one or more adjustment parameters based on the identified cross-sectional area of the first bone portion and the amount of attenuation of the joint. The determining the one or more adjustment parameters may include applying a linear equation that receives, as input, the identified cross-sectional area and the amount of attenuation of the joint, and the one or more adjustment parameters may include a predicted change in soft tissue laxity after the identified first bone portion is removed. The one or more adjustment parameters may further include an adjustment to a planned bone resection of one or more bone cuts, an adjustment to a planned bone resection angle of the one or more bone cuts, and/or an adjustment to a planned thickness of an implant. The method may further include outputting the one or more determined adjustment parameters to a display.

The one or more bone resection parameters may include a planned bone resection depth of the one or more bone cuts and/or a planned bone resection angle of the one or more bone cuts.

According to yet another aspect of the present disclosure, a system configured to assess a joint may include an image acquisition device configured to acquire at least one image of the joint, a memory configured to store information, the information including imaging data related to the at least on acquired image and pose parameters. The imaging data may include a cross-sectional area and position of at least one bone portion of the joint positioned under a soft tissue. The pose parameters may include a medial ligament in extension value, a lateral ligament in extension value, a medial ligament in flexion value, and a lateral ligament in flexion value. The system may include a controller configured to execute a first algorithm to determine a lateral ligament laxity. The first algorithm may receive, as input, the at least one image and the lateral ligament in flexion. The system may execute a second algorithm to determine, based on the lateral ligament laxity, the at least one acquired image, and/or the stored imaging data, one or more adjustment parameters. The second algorithm may apply a linear equation that receives, as input, the lateral ligament laxity and the cross-sectional area of the at least one bone portion, and outputs the one or more adjustment parameters. The one or more adjustment parameters may include an adjustment to a bone resection parameter and/or an adjustment to an implant parameter. The system may include a display configured to display the determined one or more adjustment parameters.

The image acquisition device may be a computed tomography (CT) acquisition device, and the acquired at least one image may be a CT scan.

The CT scan may show a view of the joint in a first dimension and a second dimension over which the soft tissue extends, and the cross-sectional area may be determined using the dimension of the bone portion in the first dimension and the second dimension.

The display may be configured to display a graphical user interface that may include a notification based on one or more bone portions identified in the acquired image.

### BRIEF DESCRIPTION OF THE DRAWINGS

A more complete appreciation of the subject matter of this disclosure and the various advantages thereof may be understood by reference to the following detailed description, in which reference is made to the following accompanying drawings:
FIG. 1 illustrates an exemplary portion of a patient's anatomy, according to aspects of this disclosure.
FIG. 2 illustrates an exemplary knee joint in flexion and extension, according to aspects of this disclosure.
FIG. 3 illustrates a cross-sectional view of an exemplary knee joint including one or more implant components, according to aspects of this disclosure.
FIG.4 illustrates an exemplary electronic data processing system including a bone balancing system, according to an exemplary embodiment.
FIG. 5 illustrates an exemplary process flow diagram including data, inputs, and outputs of the bone balancing system of FIG. 4, according to aspects of this disclosure.
FIG. 6 illustrates a second exemplary process flow diagram including data, inputs, and outputs of the bone balancing system of FIG. 4, according to aspects of this disclosure.
FIG. 7 illustrates an exemplary graphical user interface of the bone balancing system of FIG. 4, according to aspects of this disclosure.
FIG. 8 illustrates an exemplary preoperative method for adjusting a preoperative plan or procedure plan based on detected lateral ligament laxity and bone portions, according to aspects of this disclosure.
FIG. 9 illustrates an exemplary preoperative method for adjusting a preoperative plan or procedure plan based on detected joint attenuation and bone portions, according to aspects of this disclosure.
FIG. 10A illustrates a first exemplary portion of a patient's joint, according to aspects of this disclosure.
FIG. 10B illustrates a second exemplary portion of a patient's joint, according to aspects of this disclosure.
FIG. 10C illustrates a third exemplary portion of a patient's joint, according to aspects of this disclosure.
FIG. 10D illustrates a fourth exemplary portion of a patient's joint, according to aspects of this disclosure.

### DETAILED DESCRIPTION

Reference will now be made in detail to the various embodiments of the present disclosure illustrated in the accompanying drawings. Wherever possible, the same or like reference numbers will be used throughout the drawings to refer to the same or like features. It should be noted that the drawings are in simplified form and are not drawn to precise scale. Additionally, the term "a," as used in the specification, means "at least one." The terminology includes the words above specifically mentioned, derivatives thereof, and words of similar import. Although at least two variations are described herein, other variations may include aspects described herein combined in any suitable manner having combinations of all or some of the aspects described.

As used herein, the terms "implant trial" and "trial" will be used interchangeably and as such, unless otherwise stated, the explicit use of either term is inclusive of the other term. In this disclosure, "user" is synonymous with "practitioner" and may be any person completing the described action (e.g., surgeon, technician, nurse, etc.).

An implant may be a device that is at least partially implanted in a patient and/or provided inside of a patient's body. For example, an implant may be a sensor, artificial bone, or other medical device coupled to, implanted in, and/or at least partially implanted in a bone, skin, tissue, organs, etc. A prosthesis or prosthetic may be a device configured to assist or replace a limb, bone, skin, tissue, etc., or portion thereof. Many prostheses are implants, such as a tibial prosthetic component. Some prostheses may be exposed to an exterior of the body and/or may be partially implanted, such as an artificial forearm or leg. Some prostheses may not be considered implants and/or otherwise may be fully exterior to the body, such as a knee brace. Systems and methods disclosed herein may be used in connection with implants, prostheses that are implants, and also prostheses that may not be considered to be "implants" in a strict sense. Therefore, the terms "implant" and "prosthesis" will be used interchangeably and as such, unless otherwise stated, the explicit use of either term is inclusive of the other term. Although the term "implant" is used throughout the disclosure, this term should be inclusive of prostheses which may not necessarily be "implants" in a strict sense.

In describing preferred embodiments of the disclosure, reference will be made to directional nomenclature used in describing the human body. It is noted that this nomenclature is used only for convenience and that it is not intended to be limiting with respect to the scope of the invention. For example, as used herein, the term "distal" means toward the human body and/or away from the operator, and the term "proximal" means away from the human body and/or towards the operator. As used herein, the terms "comprises," "comprising," or any other variation thereof, are intended to cover a non-exclusive inclusion, such that a process, method, article, or apparatus that comprises a list of elements does not include only those elements, but may include other elements not expressly listed or inherent to such system, process, method, article, or apparatus. The term "exemplary" is used in the sense of "example," rather than "ideal." Further, relative terms such as, for example, "about," "substantially," "approximately," etc., are used to indicate a possible variation of ±10% in a stated numeric value or range.

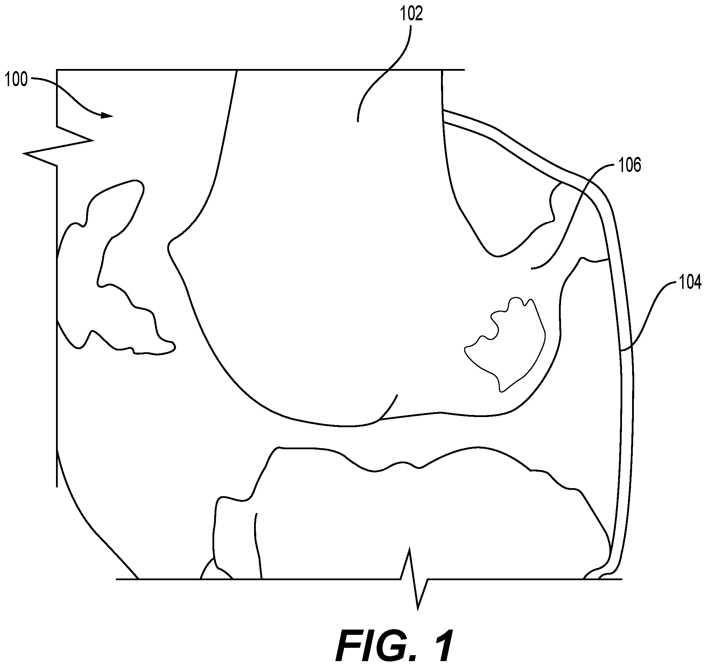

FIG. 1 illustrates an exemplary portion of anatomy 100 (e.g., leg or knee joint). Referring to FIG. 1, the portion of anatomy 100 may include one or more bones 102 (e.g., tibia or femur) and one or more soft tissues 104 (e.g., ligament). Inserting an implant during a medical procedure (e.g., total knee arthroplasty or TKA) may be desired to treat disease, reduce pain, and/or restore proper function of a knee joint. However, following the implant procedure, soft tissues may have increased slack and/or may not fully contract to an initial state, or the soft tissues may be tighter than their pre-operative state and reduce a range of motion of the joint, due to changed geometry of bones 102. Thus, this reduced or increased laxity in soft tissue may result in the joint being looser or tighter, potentially reducing the functionality of the joint and increasing patient complications.

One of ordinary skill in the art will appreciate that the medial collateral ligament and the lateral collateral ligament have differing biomechanical properties. For example, the medial collateral ligament may have clearly defined start and end points of longitudinal flexibility (or stretch), whereas the lateral collateral ligament may not have clearly defined start and end points of flexibility. The medial collateral ligament may be less elastic (e.g., have less laxity) than the lateral collateral ligament. These biomechanical differences necessitate differing treatment of these ligaments during a TKA or other joint procedures. A need exists in the field for reproducible means of determining postoperative or target lateral laxity (e.g., laxity associated with the lateral collateral ligament) in joint procedures. This disclosure focuses on a target post-procedure lateral laxity for a patient identical to the patient's specific pre-procedure lateral laxity, which has been determined to optimize procedure outcomes. Reproducible means of quantifying pre-surgical and post-surgical lateral laxity remains a need in the art. For example, a surgeon may need to determine how much native laxity exists in the lateral collateral ligament (e.g., 1-5 mm) and how much distraction force (if any) should be applied to a lateral side of anatomy 100 to determine the correct amount of lateral laxity (e.g., 20-100 lbs.).

One of ordinary skill in the art will appreciate that joint attenuation may be caused by subluxation, a translation of one bone with respect to another bone of a joint. Subluxation is associated with end-stage arthritis and can exhibit significantly varied biomechanical characteristics across patient populations. For instance, the degree of joint attenuation caused by subluxation may be influenced by the patient's underlying anatomy, ligament integrity, and surrounding soft tissues, which may each display different thresholds of flexibility or stability. The length of various ligaments, such as the MCL and/or the LCL, may be increased or otherwise stretched due to subluxation. These variations can affect the joint's range of motion, weight-bearing capacity, and susceptibility to further subluxation-induced attenuation. In an example, subluxation of the knee may be defined as translation of the tibia under the femur. Attenuation caused by subluxation may create changed characteristics in associated ligaments, which may in turn affect how a surgeon may properly balance the joint during corrective surgery. Attenuation may accordingly be one of multiple variables a surgeon must consider when balancing a joint. Medial reduction osteotomy is a surgical technique that shares similar effects on laxity as chronic subluxation. A surgeon may resect a portion of the medial tibia to create medial laxity in scenarios in which varus deformity results in excessive tightness of the medial side. The degree of laxity generated is directly related to the amount of bone resected. When the tibia is reduced under the femur, the medial side effectively lengthens, an effect that can be predicted.

Consequently, reproducible methods for assessing and quantifying the degree of joint attenuation caused by subluxation and/or bone cuts (both before and after a corrective procedure) remain important to achieving consistent and optimized patient outcomes. A need exists in the art to establish reliable methodology that accurately measures pre-procedural joint attenuation for improved surgical planning and patient outcomes. For example, a surgeon may determine the native attenuation profile-and apply precisely measured corrective forces to restore or maintain joint alignment without overcorrecting or compromising the surrounding anatomical structures (e.g., from excessive tissue cuts necessitating use of excessively large spacers).

Aspects disclosed herein may be configured to optimize a "fit" or "tightness" of an implant provided to a patient during a medical procedure based on detections by the one or more algorithms related to a lateral laxity in a lateral compartment and or attenuation of a joint. A fit of the implant may be made tighter by aligning the implant with a shallower bone slope and/or determining a shallower resulting or desired bone slope, by increasing a thickness or other dimensions of the implant, by determining certain types of materials or a type of implants or prosthesis (e.g., a stabilizing implant, a VVC implant, an ADM implant, or an MDM implant). A thickness of the implant may be achieved by increasing (or decrease) a size or shape of the implant. Tightness may be impacted by gaps and/or joint space width, which may be regulated by an insert which may vary depending on a type of implant or due to a motion. Gaps may be impacted by femoral and tibial cuts, among other parameters. Tightness may further be impacted by slope. A range of slope may be based on implant choice as well as surgical approach and patient anatomy. A thickness of the implant may also be achieved by adding or removing an augment or shim. For example, augments or shims may be stackable and removable, and a thickness may be increased by adding one or more augments or shims or adding an augment or shim having a predetermined (e.g., above a certain threshold) thickness. Fit or tightness may also be achieved with certain types of bone cuts, bone preparations, or tissue cuts that reduce a number of cuts made and/or an invasiveness during surgery.

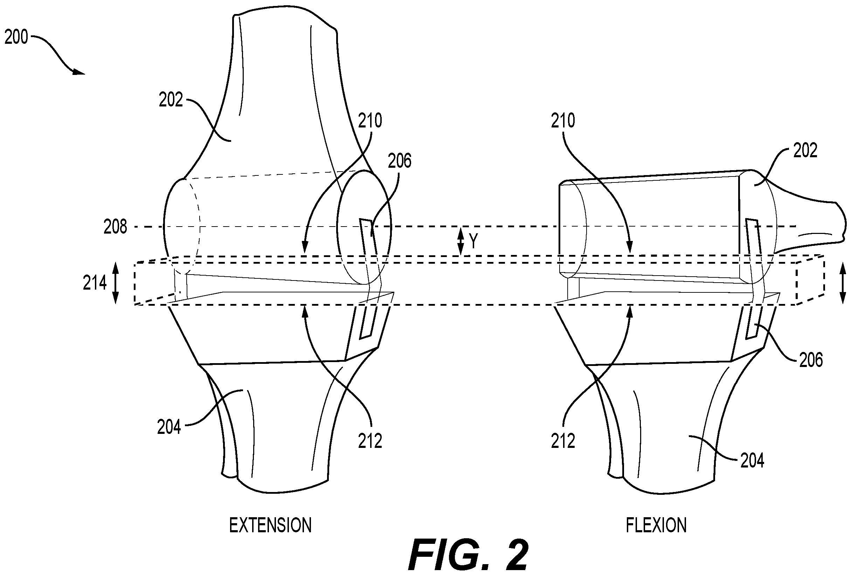

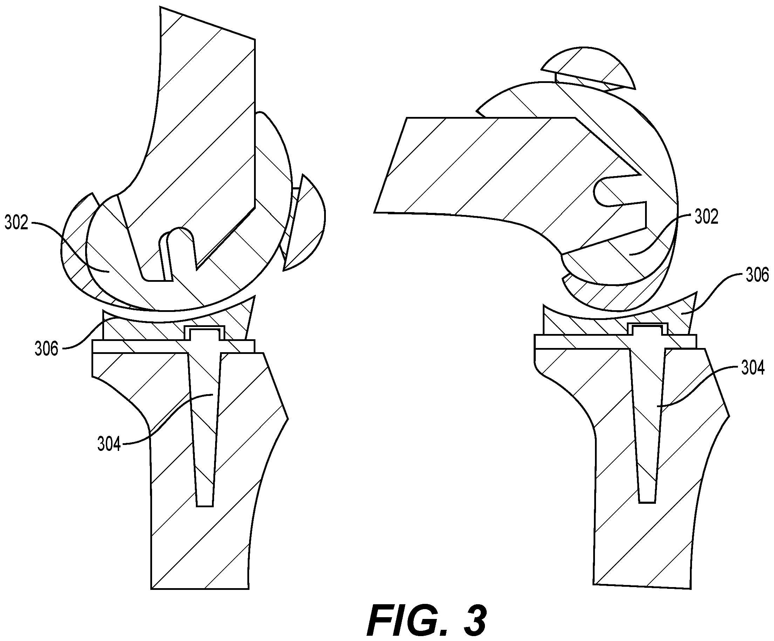

FIG. 2 illustrates an exemplary knee joint 200 in flexion and extension. Referring to FIG. 2, the knee joint 200 may include a femur 202, a tibia 204, a ligament 206 (e.g., medial collateral ligament), and a ligament 218 (e.g., a lateral collateral ligament) coupled to the femur 202 and the tibia 204. The femur 202 may rotate with respect to tibia 204 about a flexion-extension axis 208 extending through the femur 202. A procedure plan (e.g., surgery plan or medical operation plan) may include one or more bone cuts, such as a distal and posterior femoral resection 210 through the femur 202 and/or a proximal tibial resection 212 through the tibia 204. When the knee joint 200 is in extension, an extension gap 214 may extend between the femoral resection 210 and the tibial resection 212. When the knee joint 200 is in flexion, a flexion gap 216 may extend between the femoral resection 210 and the tibial resection 212. The femoral resection 210 and the tibial resection 212 may be configured to provide a balanced knee joint 200, and in some examples, in conjunction with one or more prosthetic components or liners such as the femoral prosthetic component 302, the tibial prosthetic component 304, and the liner 306 shown in FIG. 3.

When planning for a medical procedure at the knee joint 200, a practitioner (e.g., surgeon, doctor, healthcare planner, etc.) may desire to make the extension gap 214 equal to the flexion gap 216. In some examples, the practitioner may plan to create a rectangular flexion gap 216 by adjusting a femoral cutting block so that the femoral cutting block is parallel to a resected tibial surface at 90° with the ligaments (e.g., ligament 206) under tension. These adjustments and the laxity of the ligament 218, as explained above, are affected by the implant and various cuts, among other factors. Thus, the practitioner may wish to determine the lateral laxity prior to planning (or fine tuning of the plan) and making bone cuts. Thus, for gap balancing, a practitioner may try to predict the effect of various factors on the laxity of ligament 218 (e.g., the lateral laxity) before any bone cuts are made, similar to how a golfer may make an adjustment in aim to compensate for prevailing wind.

Aspects disclosed herein may analyze a lateral laxity to determine a dimension and/or design of one or more implants or prosthetics used in a procedure, such a thickness, size, material, and/or shape of the femoral prosthetic component 302, the tibial prosthetic component 304, and/or the liner 306 shown in FIG. 3 such that post-surgical lateral laxity is substantially similar to pre-surgical lateral laxity.

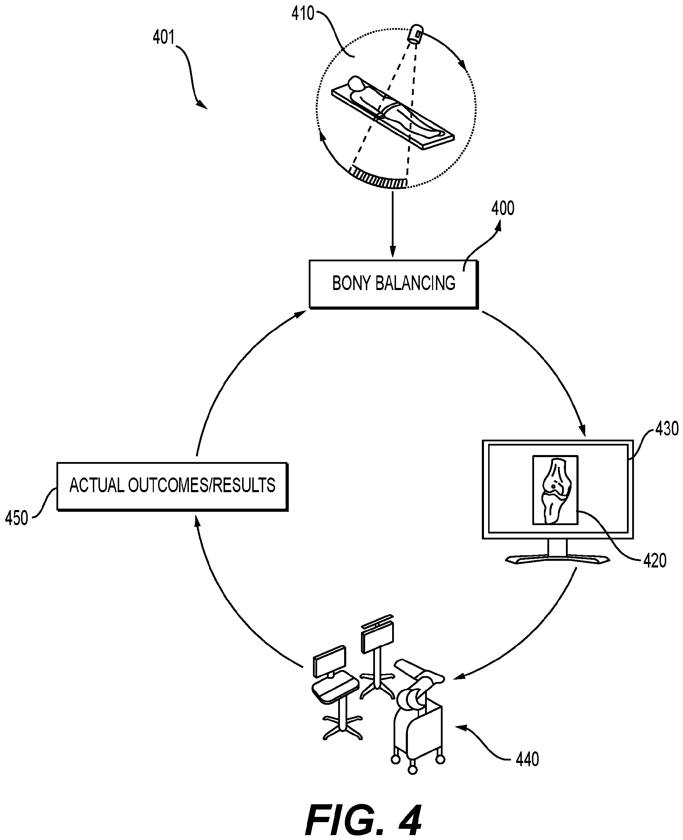

Referring to FIG. 4, an electronic data processing system 401 may include a bony balancing system 400 including one or more algorithms. The bony balancing system 400 may receive one or more medical images of patient's anatomy acquired using one or more image acquisition devices 410, analyze the received one or more medical images to determine and/or adjust a procedure plan 420 (including a patient-specific lateral laxity), which may be output into a display 430 and/or to a robotic and/or automated data system or platform 440 (e.g., a robotic system such as a surgical robot and/or a robotic tool). Results and/or outcome data 450 from the procedure may be fed to the bony balancing system 400 for further refinement of the one or more algorithms (e.g., a patient-specific gap balancing algorithm).

The bony balancing system 400 may be implemented as one or more computer systems or cloud-based electronic processing systems. The image acquisition device 410 may include a computed tomography (CT) scanner, a magnetic resonance imaging (MRI) machine, an x-ray machine, a radiography system, an ultrasound system, a thermography system, a tactile imaging system, an electrography, nuclear medicine functional imaging system, a positron emission tomography (PET) system, a single-photon emission computer tomography (SPECT) system, a camera, etc. An instant patient who is planning to undergo a procedure (e.g., surgery) may first undergo imaging using the image acquisition device 410 (e.g., a CT scanner). Images and/or information collected during imaging (e.g., CT or CAT scans) may be transmitted from or stored in the image acquisition device 410.

During imaging using the image acquisition device 410, the patient may undergo one or more "poses" or positions where certain soft tissues of a joint (e.g., medial and/or lateral soft tissues) are stressed in certain positions of the joint (e.g., flexion and extension) by applying one or more forces (e.g., varus and valgus forces). The bony balancing system 400 may determine and/or calculate a size and/or shape of one or more gaps of the joint (e.g., flexion and extension gaps) to assess the soft tissue envelope of the joint. Alternatively or in addition thereto, the practitioner may determine a size and/or shape of the one or more gaps and input the determined size and/or shape into the bony balancing system 400 (e.g., via a user interface such as display 430). The bony balancing system 400 may identify bone landmarks from the images and/or data collected at the poses. Alternatively, a practitioner may analyze the images and input, into the bony balancing system 400, imaging data, such as positions and dimensions of bone landmarks.

The bony balancing system 400 may execute the one or more algorithms, using the imaging data including the determined gap (e.g., flexion and extension gap), the lateral laxity, and/or the identified bone landmarks to determine the procedure plan 420. The procedure plan 420 may include a series of steps to perform in the procedure, such as one or more bone resection parameters (e.g., resections or cuts to make in a bone) and/or a design of an implant (e.g., prosthetic liner). The procedure plan 420 may also include predicted outcomes (e.g., a risk of complication during the procedure or a risk of infection post-procedure). As an example, the procedure plan 420 may first be determined based on the size and/or the shape of the one or more gaps of the joint and then may be revised based on changing lateral laxity during or after the procedure.

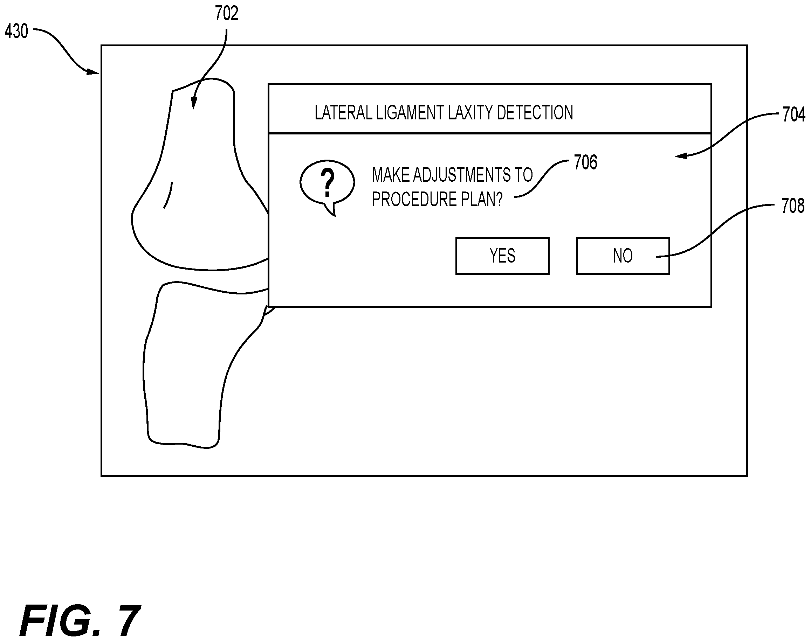

In some examples, the procedure plan 420, including the one or more bone resection parameters 562, and/or implant parameters 564 (FIG. 7), may be transmitted to a display 430. The one or more bone parameters 562 and the one or more implant parameters 564 of the procedure plan 420 may collectively be referred to as one or more adjustment parameters. In some examples, the procedure plan 420, including the one or more bone resection parameters 562 and/or implant parameters 564, may be transmitted to a surgical robot or robotic tool (e.g., an automated cutting burr) of the robotic and/or automated data system 440 to execute the determined bone resection parameters 562 by, for example, automatically cutting, via a surgical robot holding a tool, a surgeon holding a robotic tool, etc., a bone according to the bone resection parameters 562 of the procedure plan 420. As the course of treatment is continued, the actual or observed outcomes and/or results 450 may also be used by the bony balancing system 400 to either update its predictions (e.g., intraoperatively based on intraoperatively collected data or outcomes) and/or to make future predictions for future patients (e.g., based on a postoperative result or outcome). Intraoperative data for further refinements may be similar to the preoperative data 520. Details of the bony balancing system 400 and its determinations will be described in more detail with respect to FIG. 7.

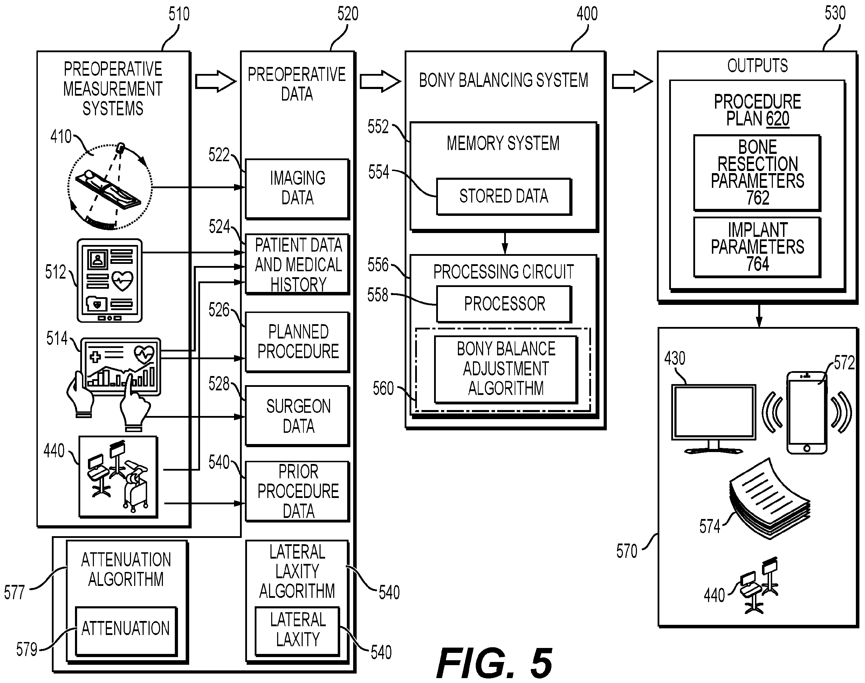

FIG. 5 illustrates exemplary data, inputs, and outputs of the bony balancing system 400. Referring to FIG. 5, the bony balancing system 400 may receive preoperative data 520 from one or more preoperative measurement systems 510 and analyze the preoperative data 520 to produce one or more outputs 530 such as the procedure plan 420 to one or more output systems 570, such as the display 430. The preoperative measurement systems 510 may include the image acquisition device 410; electronic devices storing electronic medical records (EMR) 514; patient, practitioner, and/or user interfaces or applications 750 (such as on tablets, computers, or other mobile devices); and the robotic and/or automated data system or platform 440 (e.g., MAKO Robot System or platform, MakoSuite, etc.), which may include a robotic device, as previously mentioned.

The bony balancing system 400 may receive imaging data 522 via the image acquisition device 410 and supplemental or additional information (e.g., patient data and medical history 524, planned procedure data 526, surgeon and/or staff data 528, and/or prior procedure data 540) via EMR 512, interfaces 514, sensors and/or electronic medical devices, and/or robotic platform 440. Each of the devices in the preoperative measurement systems 510 (the image acquisition device 410, EMR 512, user interfaces or applications 514, sensors and/or electronic medical devices, and robotic platform 440) may include one or more communication modules (e.g., Wi-Fi modules, Bluetooth modules, etc.) configured to transmit preoperative data 520 to each other, to the bony balancing system 400, and/or to the one or more output systems 570.

The image acquisition device 410 may be configured to collect or acquire one or more images, videos, or scans of a patient's internal anatomy, such as bones, ligaments, soft tissues, brain tissue, etc. to provide imaging data 522, which will be described in more detail later. As previously described, the image acquisition device 410 may include a computed tomography (CT) scanner, a magnetic resonance imaging (MRI) machine, an x-ray machine, a radiography system, an ultrasound system, a thermography system, a tactile imaging system, an electrography, nuclear medicine functional imaging system, a positron emission tomography (PET) system, a single-photon emission computer tomography (SPECT) system, a camera, etc. The collected images, videos, or scans may be transmitted, automatically or manually, to the bony balancing system 400. In some examples, a user may select specific images from a plurality of images taken with the image acquisition device 410 to be transmitted to the bony balancing system 400.

The bony balancing system 400 may use previously collected data from EMR 512, which may include patient data and medical history 524 in the form of past practitioner assessments, medical records, past patient reported data, past imaging procedures, treatments, etc. For example, EMR 512 may contain data on demographics, medical history, biometrics, past procedures, general observations about the patient (e.g., mental health), lifestyle information, data from physical therapy, etc.

The bony balancing system 400 may also use present or current (e.g., in real time) patient data via patient, practitioner, and/or user interfaces or applications 514. These user interfaces 514 may be implemented on mobile applications and/or patient management websites or interfaces, such as OrthologIQ^{®}. User interfaces 514 may present questionnaires, surveys, or other prompts for practitioners or patients to enter assessments (e.g., throughout a prehabilitation program prior to a procedure), observed data and/or reactions (e.g., in response to various poses), psychosocial information and/or readiness for surgery, comments, etc. for additional patient data 524. Patients may also enter psychosocial information such as perceived or evaluated pain, stress level, anxiety level, feelings, and other patient reported outcome measures (PROMS) into these user interfaces 514. Patients and/or practitioners may report lifestyle information via user interfaces 514. User interfaces 514 may also collect clinical data such as planned procedure 526 data and planned surgeon and/or staff data 528 described in more detail later. These user interfaces 514 may be executed on and/or combined with other devices disclosed herein (e.g., with robotic platform 440).

The bony balancing system 400 may receive prior procedure data 540 from prior patients and/or other real-time data or observations (e.g., observed patient data 524) via robotic platform 440. The robotic platform 440 may include one or more robotic devices (e.g., surgical robot), computers, databases, etc. used in prior procedures with different patients. The robotic platform 440 may have assisted with, via automated movement, surgeon assisted movement, and/or sensing, a prior procedure and may be implemented as or include one or more automated or robotic surgical tools, robotic surgical or Computerized Numerical Control (CNC) robots, surgical haptic robots, surgical tele-operative robots, surgical hand-held robots, or any other surgical robot.

Although the preoperative measurement system(s) 510 is described in connection with image acquisition device 410, EMR 512, user interfaces 514, and robotic platform 440, other devices may be used preoperatively to collect preoperative data 520, for example data relating to joint alignment and/or identified bone landmarks or other data sued to create procedure plan 420. For example, mobile devices such as cell phones and/or smart watches may include various sensors (e.g., gyroscopes, accelerometers, temperature sensors, optical or light sensors, magnetometer, compass, global positioning systems (GPS) etc.) to collect patient data 524 such as location data, sleep patterns, movement data, heart rate data, lifestyle data, activity data, etc. As another example, wearable sensors, heart rate monitors, motion sensors, external cameras, etc. having various sensors (e.g., cameras, optical light sensors, barometers, GPS, accelerometers, temperature sensors, pressure sensors, magnetometer or compass, MEMs devices, inclinometers, acoustical ranging, etc.) may be used during physical therapy or a prehabilitation program to collect information on patient kinematics, alignment, movement, fitness, heart rate, electrocardiogram data, breathing rate, temperature, oxygenation, sleep patterns, activity frequency and intensity, sweat, perspiration, air circulation, stress, step pressure or push-off power, balance, heel strike, gait, fall risk, frailty, overall function, etc. Other types of systems or devices may include electromyography or EMG systems or devices, motion capture (mocap) systems, sensors using machine vision (MV) technology, virtual reality (VR) or augmented reality (AR) systems, etc.

The preoperative data 520 may be data collected, received, and/or stored prior to an initiation of a medical treatment plan or medical procedure. As shown by the arrows in FIG. 5, the preoperative data 520 may be collected using the preoperative measurement systems 510, from a memory system 552 (e.g., cloud storage system) of the bony balancing system 400, and from output systems 570 (e.g., from a prior procedure) for one or more continuous feedback loops. Some of the preoperative data 524 may be directly sensed via one or more devices (e.g., image acquisition device 410 and/or wearable motion sensors or mobile devices) or may be manually entered by a medical professional, patient, or other party. Other preoperative data 524 may be determined (e.g., by bony balancing system 400) based on directly sensed information, input information, and/or stored information from prior medical procedures.

As previously described, the preoperative data 524 may include imaging data 522, patient data and/or medical history 524, information on a planned procedure 526, surgeon data 528, and prior procedure data 540.

The imaging data 522 may include one or more images (e.g., raw images), videos, or scans of a patient's anatomy collected and/or acquired by the image acquisition device 410. The bony balancing system 400 may receive and analyze one or more of these images to determine further imaging data 522, which may be used as further input preoperative data 524. In some examples, image acquisition device 410 may analyze and/or process the one or more images and send any analyzed and/or processed imaging data to the bony balancing system 400 for further analysis.

The one or more images of the imaging data 522 may illustrate or indicate, and the bony balancing system 400 may be configured to identify and/or recognize in the images: bone, soft tissue, or cartilage positions or alignment, composition and/or density, fractures and/or tears, bone landmarks (e.g., condyle surface, head or epiphysis, neck or metaphysis, body or diaphysis, articular surface, epicondyle, lateral epicondyle, medial epicondyle, process, protuberance, tubercle vs tuberosity, tibial tubercle, trochanter, spine, linea or line, facet, crests and ridges, foramen and fissure, meatus, fossa and fovea, incisure and sulcus, and sinus), geometry (e.g., diameters, slopes, angles) and/or other anatomical geometry data such as deformities or flare (e.g., coronal plane deformity, sagittal plane deformity, lateral femoral metaphyseal flare, or medial femoral metaphyseal flare). Such geometry is not limited to overall geometry and may include relative dimensions (e.g., lengths or thicknesses of a tibia or femur).

The imaging data 522 may include information about an identified or detected joint, such as a knee joint. The imaging data 522 may include dimensions and/or positioning of the joint. Analysis and/or calculations that may be derived from the images or scans will be described in more detail later when describing the bony balancing system 400.

In addition, the imaging data 522 may include morphology and/or anthropometrics (e.g., physical dimensions of internal organs, bones, etc.), fractures, slope or angular data, tibial slope, posterior tibial slope or PTS, bone density, (e.g., bone mineral or bone marrow density, bone softness or hardness, or bone impact), etc. Imaging data 522 may not be limited to bone data and may be inclusive of other internal imaging data, such as cartilage, soft tissue, blood flow, or ligaments.

In addition to raw images, imaging data 522 may include intermediate and/or related imaging data 522 to be used by the bony balancing system 400 to calculate outputs 530. Such intermediate imaging data 522 may include density or composition charts or graphs; quantified data indicating relative positions, dimensions, etc.; and/or processed image data indicating specifically detected attributes, such as a probability of a certain patient condition. In some examples, intermediate imaging data 522 may include a laxity associated with soft tissue, such as a ligament. One or more algorithms 560 of the bony balancing system 400 may determine or calculate this intermediate imaging data 522 in determining outputs 530, or alternatively or additionally thereto, the image acquisition device 410 may include one or more processors configured to calculate or quantify, based on the raw images, videos, or scans, at least some of the intermediate imaging data 522. Intermediate imaging data 522 may include information relating to, indicating, and/or quantifying aspects of the raw images, charts, etc.

Patient data and medical history 524 may include information about the instant patient on identity (e.g., name or birthdate), demographics (e.g., patient age, gender, height, weight, nationality, body mass index (BMI), etc.), lifestyle (e.g., smoking habits, exercise habits, drinking habits, eating habits, fitness, activity level, frequency of climbing activities such as up and down stairs, frequency of sit-to-stand movements or bending movements such as when entering and exiting a vehicle, steps per day, activities of daily living or ADLs performed, etc.), medical history (e.g., allergies, disease progressions, addictions, prior medication use, prior drug use, prior infections, frailties, comorbidities, prior surgeries or treatment, prior injuries, prior pregnancies, utilization of orthotics, braces, prosthetics, or other medical devices, etc.), assessments and/or evaluations (e.g., laboratory tests and/or bloodwork, American Society of Anesthesiology or ASA score and/or fitness for surgery or aesthesia) electromyography data (muscle response or electrical activity in response to a nerve's stimulation), psychosocial information (e.g., perceived pain, stress level, anxiety level, mental health status, PROMS (e.g., knee injury and osteoarthritis outcome score or KOOS, hip disability and osteoarthritis outcome score or HOOS, pain virtual analog scale or VAS, PROMIS Global 10 or PROMIS-10, EQ-5D, a mental component summary, satisfaction or expectation information, etc.), past biometrics (e.g., heart rate or heat rate variability, electrocardiogram data, breathing rate, temperature (e.g., internal or skin temperature), fingerprints, DNA, etc.), past kinematics or alignment data, past imaging data, data from prehabilitation programs or physical therapy (e.g., average load bearing time) etc. Medical history 524 may include prior clinical or hospital visit information, including encounter types, dates of admission, hospital-reported comorbidity data such as Elixhauser and/or Charlson scores or selected comorbidities (e.g., ICD-10 POA), prior anesthesia taken and/or reactions, etc. This list, however, is not exhaustive and preoperative data 520 may include other patient specific information, clinical information, and/or surgeon or practitioner specific information (e.g., experience level).

Patient data 524 may come from EMR 512, from user interfaces 514, from memory system 552, and/or from robotic platform 440, but aspects disclosed herein are not limited to a collection of the patient data 524. For example, other types of patient data 524 or additional data may include data on activity level; kinematics; muscle function or capability; range of motion data; strength measurements and/or force measurements push-off power, force, or acceleration; a power, force, or acceleration at a toe during walking; angular range or axes of joint motion or joint range of motion; flexion or extension data, including step data (e.g., measured by a pedometer), gait data or assessments; fall risk data; balancing data; joint stiffness or laxity data; postural sway data; data from tests conducted in a clinic or remotely; etc.

Information on a planned procedure 526 may include an initial procedure plan 420 and/or logistical information about the procedure and substantive information about the procedure. Substantive planned procedure 526 information may include a surgeon's surgical or other procedure or treatment plan, including planned steps or instructions on incisions, a side of the patient's body to operate on (e.g., left or right) and/or laterality information, bone cuts or resection depths, implant design, type, and/or size, implant alignment, fixation or tool information (e.g., implants, rods, plates, screws, wires, nails, bearings used), cementing versus cement-less techniques or implants, final or desired alignment, pose or orientation information (e.g., capture gap values for flexion or extension, gap space or width between two or more bones, joint alignment), planning time, gap balancing time, extended haptic boundary usage, etc. Logistical planned procedure 526 information may include information about a planned site of the procedure such as a hospital, a type of procedure or surgery to be performed (e.g., total or partial knee arthroplasty or replacement, total or partial hip arthroplasty or replacement, spine surgery, patella resurfacing), scheduling or booking information, equipment or tools required, etc. This initial planned procedure 526 information may be manually prepared or input by a surgeon and/or previously prepared or determined using one or more algorithms. Surgeon data 528 may include information about a surgeon or other staff planned to perform the procedure plan 420 such as identity (e.g., name), experience level, fitness level, height, and/or weight, etc.

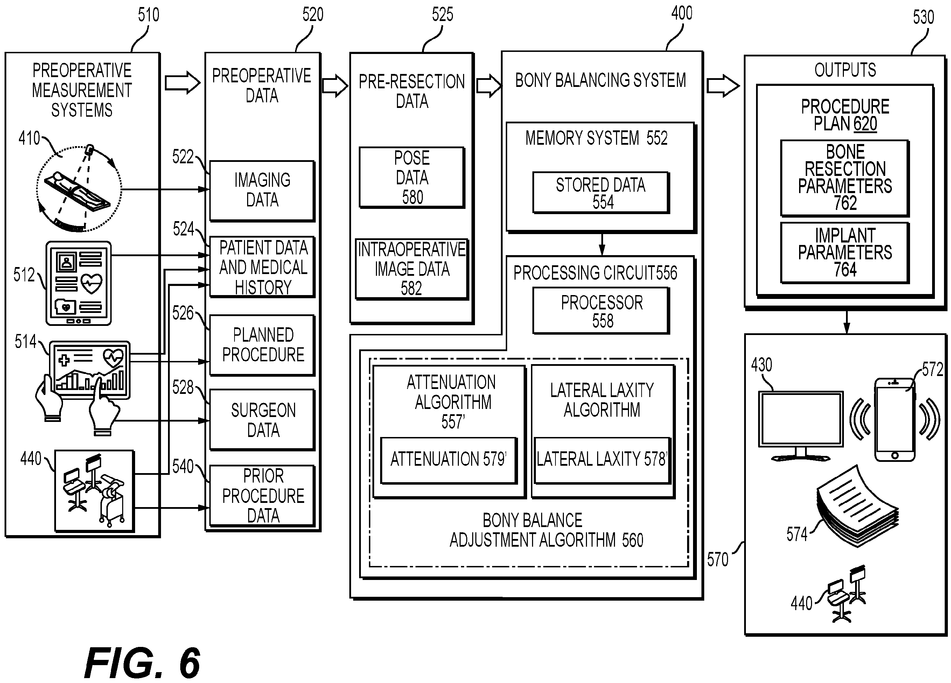

Prior procedure data 540 may include information about prior procedures performed on a same or prior patient. Such information may include the same type of information as in planned procedure data 528 (e.g., instructions or steps of a procedure, bone cuts, implant design, implant alignment, etc.) along with outcome and/or result information (e.g., outcomes 450 described with reference to FIG. 6), which may include both immediate results and long-term results, complications after surgery, length of stay in a hospital, revision surgery data, rehabilitation data, patient motion and/or movement data, etc. Prior procedure data 540 may include information about prior procedures of prior patients sharing at least one same or similar characteristic (e.g., demographically, biometrically, disease state, etc.) as the instant patient.

Preoperative data 520 may include a lateral laxity 578, which may be an output of a lateral laxity algorithm 576. Lateral laxity algorithm 576 may require up to four inputs: (1) medial extension (e.g., valgus stress test), (2) lateral extension (e.g., varus stress test), (3) medial in flexion at the asymptote (e.g., endpoint) of stress, and (4) lateral side at rest (e.g., in flexion). The four inputs may also be referred to as pose capture data. In some examples, only one, two, or three of these four inputs may be required. The medial extension value (1) and the lateral extension value (2) may be determined by mechanical stress testing (e.g., valgus and varus stress testing) of the medial and lateral joints. With respect to inputs (3) and (4), one of ordinary skill in the art will appreciate that varus and valgus stress testing may not be possible due to internal and external rotation of a patient's hip. One of ordinary skill in the art will appreciate that a varus stress test involves bending a patient's relaxed leg to approximately 10-30° of flexion while palpating the lateral joint line. The operator may then apply a varus force to the knee without inducing hip rotation. A positive indication may correspond to patient pain or gapping. The varus stress test may be repeated with the knee in a neutral position, 0° of flexion. A positive indication may correspond to patient pain or gapping. One of ordinary skill in the art will appreciate that valgus stress testing may be performed in a substantially similar manner to varus stress testing, but with a valgus force applied instead of a varus force. Input (3) may be determined by identification of the asymptote and/or endpoint by applying either a manual or a digital (electronic) distraction force. Input (4) may be identified with a manual distraction force or a digital tensor, or with no distraction force. Input (4) may be determined either without any distraction force applied to the joint on either the medial or lateral side, or may be determined while a distraction force is applied to the medial side such that the medial ligament is at its asymptote and/or endpoint in flexion, as described hereinabove. This technique may facilitate uncertainties around how to determine a target lateral ligament laxity, since the lateral compartment in flexion does not have a firm endpoint or asymptote and simplify the gap balancing processes for a specific patient's knee. For example, this may eliminate the need to apply a distraction force to the lateral side and eliminate any risk of over-application of a distraction force to the lateral ligaments. Each patient may be a unique lateral ligament laxity, and adjusting the gaping using the specific patient's lateral ligament laxity may result in better surgical outcomes, less patient complications, technique reproducibility across practitioners and a diverse patient anatomy, and practitioner clarity on how to approach the lateral side ligaments.

Lateral laxity algorithm 576 may be used to balance the medial side in flexion to a distinct end point. This end point may correlate with a maximum elongation of the medial collateral ligament. Lateral laxity algorithm 576 may be used to generate a lateral laxity in flexion 578 that may ultimately be used in bony balance adjustment algorithm 560. Bony balance adjustment algorithm may incorporate lateral laxity 578 such that a surgeon may reproduce the patient's "native" (e.g., pre-surgical) lateral laxity in flexion. One of ordinary skill in the art will appreciate that lateral laxity varies from patient to patient, and thus there is no universal value for a lateral laxity. Thus, lateral laxity algorithm 576 enables a surgeon to generate lateral laxities 578 across various patients and appropriately plan for a post-operative lateral laxity (in flexion) 578 that is the same as the patient's determined pre-operative lateral laxity (in flexion).

Brief reference is now made to FIG. 6. In some aspects, bony balancing system 400 may receive pre-resection data 525. Pre-resection data 525 may include pose data 580. Pose data 580 may include data from inputs: (1) medial extension (e.g., stress test), (2) lateral extension (e.g., stress test), (3) medial flexion at the asymptote (e.g., endpoint) of stress, corresponding to maximum elongation of the MCL, and (4) lateral side at rest (e.g., in flexion). These inputs may be determined as described above. Pre-resection data 525 may include intraoperative image data 582 such as one or more CT scans or X-rays. Intraoperative image data 582 may be captured before surgery begins, for example, while a patient is awaiting surgery on an operating table. It will be understood that while pre-resection data 525 shows only pose data 580 and intraoperative image data 582, various other types of data may be incorporated into pre-resection data 525, such as additional patient data 524 that is collected temporally closer to the planned medical procedure (e.g., patient stress data collected the day of the planned surgery). In the exemplary FIG. 6, lateral laxity algorithm 576', lateral laxity 578', attenuation algorithm 577', and attenuation 579' are incorporated directly into bony balance adjustment algorithm 560. Lateral laxity algorithm 576' may use pose data 580 and intraoperative image data 582 to calculate a lateral laxity 578'. Bony balance adjustment algorithm 560 may incorporate lateral laxity 578' as a metric to balance towards, such that the post-operative lateral laxity is as close as possible (e.g., approximately equal to) to pre-operative lateral laxity 578'.

Returning now to FIG. 5, Preoperative data 520 may include any other additional or supplemental information stored in memory system 552, which may also include known data and/or data from third parties, such as data from the Knee Society Clinical Rating System (KSS) or data from the Western Ontario and McMaster Universities Osteoarthritis Index (WOMAC). In addition, in some examples, intraoperative may be collected and/or received by the bony balancing system 400 to further generate and/or refine the outputs 530 (e.g., procedure plan 420).

Preoperative data 520 may include an attenuation 579 (e.g., a quantified value of total attenuation of a ligament and/or a joint), which may be an output of an attenuation algorithm 577. Attenuation algorithm 577 may require up to two inputs: (1) the original length of the MCL, and (2) the amount of subluxation. The two inputs may also be referred to as attenuation capture data. In some aspects, the original length of the MCL (and/or LCL) may be a previously known or determined value. In some aspects, identification of the original length of the MCL and/or ACL may include referencing a predetermined value.

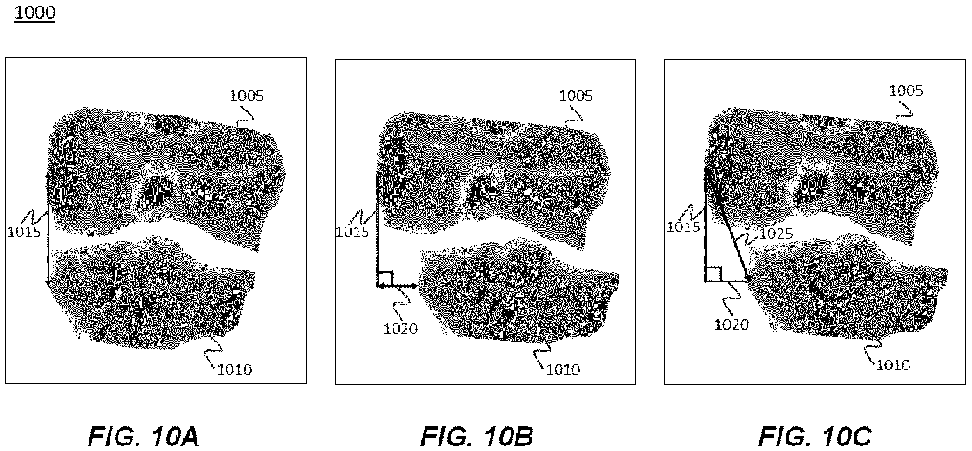

Brief reference is now made to FIGs. 10A-10C, which show an imaging scene 1000 of a patient's joint generated by a CT scan. The joint shown in FIGs. 10A-10C shows significant subluxation. A femur 1005 and a tibia 1010 are shown in FIGs. 10A-10C. FIG. 10A shows an MCL measurement 1015 of approximately 42.7 mm. FIG. 10B shows a measurement 1020 of subluxation (e.g., lateral translation) of the tibia relative to the femur of approximately 11.0 mm. In some aspects, the lengths of the MCL and/or LCL (e.g., MCL measurement 1015) may be assumed based on predetermined measurement or value, such as an averaged known value for the corresponding ligament. FIG. 10C depicts an unknown distance 1025a, which corresponds to an amount of attenuation (e.g., lengthening) of the MCL. Determination of the unknown distance 1025a is thus useful, because such a determination may be applied in conjunction with other various systems, devices, and methods disclosed herein, such as bony balance adjustment algorithm 560. As noted above, attenuation algorithm 577 may generate an attenuation value 579 based on the attenuation algorithm 577 being applied to attenuation capture data, such as measurements 1015 and 1020. The attenuation value 579 may thus equal the unknown distance 1025a. Accordingly, unknown distance 1025 (e.g., attenuation value 579) may allow a surgeon to more accurately determine their surgical plan and minimize the amount of tissue and/or bone cuts needed to balance the joint. One of ordinary skill in the art will appreciate that the measurements and specifically named bones and joints are exemplary, and that attenuation algorithm may be used with other joints, ligaments, and bones in the coronal and sagittal planes.

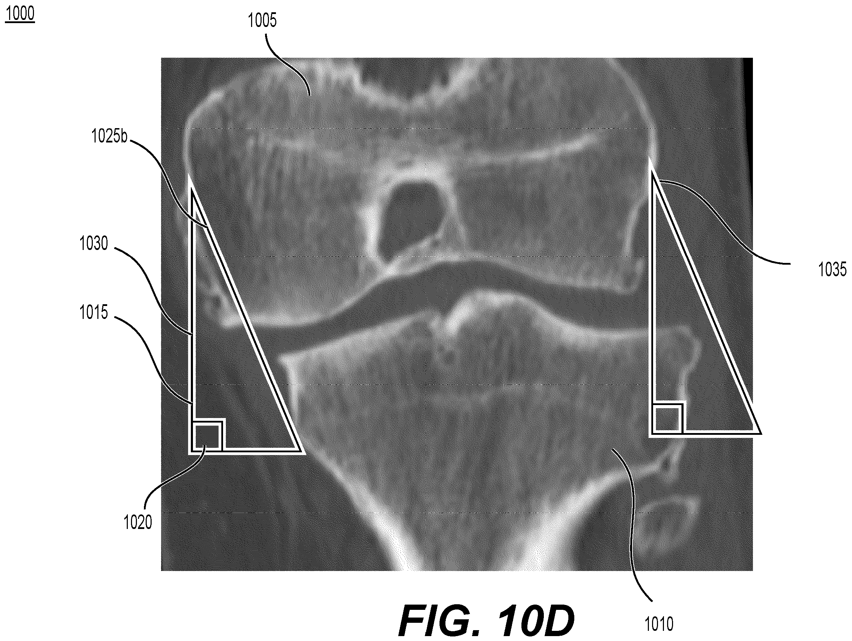

FIG. 10D illustrates the patient's joint for use in determination of attenuation of the joint, according to aspects of this disclosure. Attenuation algorithm 577 may apply one or more trigonometric techniques, such as the Pythagorean theorem, to measurements 1015 and 1020 (e.g., attenuation capture data) to determine the length of unknown distance 1025a. The triangle 1030 (e.g., a right scalene triangle) formed by measurements 1015, 1020, and unknown distance 1025a may thus be further determined via trigonometry. The determined distance of unknown distance 1025a is shown in FIG. 10D as distance 1025b, which may also be used as the value for attenuation 579. In a similar vein, a triangle 1035 may also be calculated for the opposite side of the joint as triangle 1030, for example, via similar measurement techniques applied to the lateral collateral ligament (LCL).

Predicting the amount of soft tissue laxity that may occur once the subluxed tibia is reduced can significantly aid in surgical planning. In various examples for the MCL, 6.6 mm of subluxation results in .5 mm of global laxity, 9.4 mm of subluxation results in 1 mm of global laxity, 11.4 mm of subluxation results in 1.5 mm of global laxity, 13.2 mm of subluxation results in 2 mm of global laxity, 14.8 mm of subluxation results in 2.5 mm of global laxity, 16.3 mm of subluxation results in 3 mm of global laxity, 17.6 mm of subluxation results in 3.5 mm of global laxity, 18.9 mm of subluxation results in 4 mm of global laxity, 20.2 mm of subluxation results in 4.5 mm of global laxity, and 21.3 mm of subluxation results in 5.0 mm of global laxity.

This global laxity may be effectively addressed by reducing the amount of tibial resection: specifically, for 6.6mm of subluxation, 0.5 mm less tibia may be resected, for 9.4 mm of subluxation, 1 mm less tibia may be resected, for 11.4 mm of subluxation, 1.5 mm less tibia may be resected, for 13.2 mm of subluxation, 2 mm less tibia may be resected, for 14.8 mm of subluxation, 2.5 mm less tibia may be resected, for 16.3 mm of subluxation, 3 mm less tibia may be resected, for 17.6 mm of subluxation, 3.5 mm less tibia may resected, for 18.9 mm of subluxation, 4 mm less tibia may be resected, for 20.2 mm of subluxation, 4.5 mm less tibia may be resected, and for 21.3 mm of subluxation, 5 mm less tibia may be resected.

In various examples for the LCL, 8.3 mm of subluxation results in .5 mm of global laxity, 12 mm of subluxation in 1 mm of global laxity, 14.4 mm of subluxation results in 1.5 mm of global laxity, 17 mm of subluxation results in 2 mm of global laxity, 19 mm of subluxation results in 2.5 mm of global laxity, 20.8 mm of subluxation results in 3 mm of global laxity, 22.2 mm of subluxation results in 3.5 mm of global laxity, 24 mm of subluxation results in 4 mm of global laxity, 25.3 mm of subluxation results in 4.5 mm of global laxity, 26.7 mm of subluxation results in 5.0 mm of global laxity.

The adjustment in tibial bone cuts may be performed following bony balancing determinations, which may enable calculation of the difference between native ligament length and attenuated ligament length.

Additionally, the process of predicting laxity induced by medial reduction osteotomy involves use of (or determining) the original length of the Medial Collateral Ligament (MCL) and measuring the width of the reduction osteotomy fragment while assuming a right-angle triangle configuration with one angle equal to 90 degrees. The Pythagorean theorem, expressed as Hypotenuse² = Perpendicular² + Base², is used to calculate the hypotenuse, thereby providing a quantitative assessment of the elongation of the MCL. By comparing the normal length of the MCL to the elongated length once the tibia is reduced under the femur, the resulting medial laxity can be accurately predicted. This technique is applicable to both TKA patients with chronic subluxation and those undergoing intraoperative medial reduction osteotomy. One of ordinary skill in the art will appreciate that a similar trigonometric technique is described herein for subluxation and is described in detail with respect to FIGs. 10A-10D.

When the tibial component is realigned with the femoral component during TKA, the medial soft tissue envelope is lengthened, and the amount of laxity generated is proportional to the amount of subluxation in chronic cases or the amount of bone resected during the osteotomy procedure. The amount of laxity created during medial reduction osteotomy or by chronic subluxation can be predicted by the Pythagorean theorem, and is exponential, not linear, and increases disproportionately with larger amounts of subluxation or bone cut during medial reduction osteotomy. Additionally, the laxity effect is more pronounced on the medial side due to the shorter length of the MCL compared to the Lateral Collateral Ligament (LCL), though this effect is partially offset by the relative elasticity of the LCL. When implants are placed, the medial side of the tibia is realigned with the femur, enabling prediction of the amount of correction in coronal plane alignment. The global laxity produced by this procedure can be addressed through reduced proximal tibial resection or by planning for use of a thicker liner.

Adjustments to the tibial bone cut are made following bony balancing determinations, which allow calculation of the difference between native ligament length and attenuated ligament length. This approach provides a reliable method for predicting and managing the amount of soft tissue laxity created during the reduction of the subluxed tibia under the femur, enhancing the precision and effectiveness of TKA procedures. Calculations related to laxity induced by medial reduction osteotomy may be used alone or in combination with subluxation (e.g., attenuation) data by subluxation algorithm 577 to determine attenuation 579. As described in greater detail below, subluxation algorithm 577 and attenuation 579 may be incorporated by bone balancing system 400 and bony balance adjustment algorithm 560.

### The Bony Balancing System 400

The bony balancing system 400 may include a memory system 552 including stored data 554 and a processing circuit 556 including a processor 558 and one or more algorithms. The one or more algorithms may include a bony balance adjustment algorithm 560, and in some aspects, lateral laxity algorithm 576'. The bony balance adjustment algorithm 560 may use one or more linear relationships to analyze the imaging data 522 to generate and/or modify the procedure plan 420. In some examples, the bony balancing system 400 may include an artificial intelligence (AI) and/or machine learning system that is "trained" or that may learn and refine the relationships, patterns, etc. between preoperative data 520, outputs 530, and actual results 450 (FIG. 6) to make determinations and/or, in some examples, to refine the bony balance adjustment algorithm 560. The bony balancing system 400 and the bony balance adjustment algorithm 560 may alternatively be referred to as a gap or soft tissue balancing system 400 or a gap or soft tissue balance adjustment algorithm 560.

The bony balancing system 400 may be implemented using one or more computing platforms, such as platforms including one or more computer systems and/or electronic cloud processing systems. Examples of one or more computing platforms may include, but are not limited to, smartphones, wearable devices, tablets, laptop computers, desktop computers, Internet of Things (IoT) devices, remote server/cloud-based computing devices, or other mobile or stationary devices. The bony balancing system 400 may also include one or more hosts or servers connected to a networked environment through wireless or wired connections. Remote platforms may be implemented in or function as base stations (which may also be referred to as Node Bs or evolved Node Bs (eNBs) or next generation node Bs (gNBs)). Remote platforms may also include web servers, mail servers, application servers, etc.

The bony balancing system 400 may include one or more communication modules (e.g., Wi-Fi or Bluetooth modules) configured to communicate with preoperative measurement systems 522, output system 570, and/or other third-party devices, etc. For example, such communication modules may include an Ethernet card and/or port for sending and receiving data via an Ethernet-based communications link or network, or a Wi-Fi transceiver for communication via a wireless communications network. Such communication modules may include wired or wireless interfaces (e.g., jacks, antennas, transmitters, receivers, transceivers, wire terminals, etc.) for conducting data communications with external sources via a direct connection or a network connection (e.g., an Internet connection, a LAN, WAN, or WLAN connection, LTE, 4G, 5G, Bluetooth, near field communication (NFC), radio frequency identifier (RFID), ultrawideband (UWB), etc.). Such communication modules may include a radio interface including filters, converters (for example, digital-to-analog converters and the like), mappers, a Fast Fourier Transform (FFT) module, and the like, to generate symbols for a transmission via one or more downlinks and to receive symbols (for example, via an uplink).

The memory system 552 may have one or more memories or storages configured to store or maintain the preoperative data 520, outputs 530, and stored data 554 from prior patients and/or prior procedures. The preoperative data 520 and outputs 530 of an instant procedure may also become stored data 554. Although the memory system 552 is illustrated close to processing circuit 556, memory system 552 may include memories or storages implemented on separate circuits, housings, devices, and/or computing platforms and in communication with bony balancing system 400, such as cloud storage systems and other remote electronic storage systems.

The memory system 552 may include one or more external or internal devices (random access memory or RAM, read only memory or ROM, Flash-memory, hard disk storage or HDD, solid state devices or SSD, static storage such as a magnetic or optical disk, other types of non-transitory machine or computer readable media, etc.) configured to store data and/or computer readable code and/or instructions that completes, executes, or facilitates various processes or instructions described herein. The memory system 552 may include volatile memory or non-volatile memory (e.g., semiconductor-based memory device, a magnetic memory device and system, an optical memory device and system, fixed memory, or removable memory). The memory system 552 may include database components, object code components, script components, or any other type of information structure to support the various activities described herein. In some aspects, the memory system 552 may be communicably connected to the processing circuit 556 and may include computer code to execute one or more processes described herein. The memory system 552 may contain a variety of modules, each capable of storing data and/or computer code related to specific types of functions.

The processing circuit 556 may include a processor 558 configured to execute or perform the one or more algorithms, including the bony balance adjustment algorithm 560, based on received data, which may include the preoperative data 520 and/or any data in the memory system 552 to determine the outputs 530. The preoperative data 520 may be received via manual input, retrieved from the memory system 552, and/or received direction from the preoperative measurement systems 522. The processor 558 may be configured to determine patterns based on the received data.

The processor 558 may be implemented as a general-purpose processor or computer, special purpose computer or processor, microprocessor, digital signal processor (DSPs), an application specific integrated circuit (ASIC), one or more field programmable gate arrays (FPGAs), a group of processing components, processor based on a multi-core processor architecture, or other suitable electronic processing components. The processor 558 may be configured to perform machine readable instructions, which may include one or more modules implemented as one or more functional logic, hardware logic, electronic circuitry, software modules, etc. In some cases, the processor 558 may be remote from one or more of the computing platforms comprising the bony balancing system 400. The processor 558 may be configured to perform one or more functions associated with the bony balancing system 400, such as precoding of antenna gain/phase parameters, encoding and decoding of individual bits forming a communication message, formatting of information, and overall control of one or more computing platforms comprising the bony balancing system 400, including processes related to management of communication resources and/or communication modules.

In some aspects, the processing circuit 556 and/or memory system 552 may contain several modules related to medical procedures, such as an input module, an analysis module, and an output module. The bony balancing system 400 need not be contained in a single housing. Rather, components of the bony balancing system 400 may be located in various different locations or even in a remote location. Components of the bony balancing system 400, including components of the processing circuit 556 and the memory system 552, may be located, for example, in components of different computers, robotic systems, devices, etc. used in surgical procedures, in remote systems (e.g. cloud service provider system and/or remote data center), etc.

The bony balancing system 400 may use the one or more algorithms, including the bony balance adjustment algorithm 560 and in some aspects, lateral laxity algorithm 576 or 576', to make intermediate determinations and to determine the one or more outputs 530. The one or more algorithms may be configured to determine or glean data from the preoperative data 520, including the imaging data 522. For example, the one or more algorithms may be configured for bone recognition, soft tissue recognition, and/or to make determinations related to the intermediate imaging data 522 previously described. The one or more algorithms may operate simultaneously and/or separately to determine the one or more outputs 530 and/or display the one or more outputs 530.

The one or more algorithms include the bony balance adjustment algorithm 560 and lateral laxity algorithm 576 and 576'. In addition, the one or more algorithms may include one or more machine learning algorithms that are trained using, for example, linear regression, random forest regression, CatBoost regression, statistical shape modelling or SSM, etc. The one or more algorithms may be continuously modified and/or refined based on actual outcomes and/or results 450 (FIG. 6). The one or more algorithms may be configured to use segmentation techniques and/or thresholding techniques on received images, videos, and/or scans of the imaging data 522 to determine the one or more outputs 530. For example, the one or more algorithms may be configured to segment an image (e.g., a CT scan) and/or threshold soft tissue or identified bone, bone landmarks, etc. The one or more algorithms may be configured to automate data extraction and/or collection upon receiving an image from the image acquisition device 410. The bony balance adjustment algorithm 560 will be described in more detail hereinafter.

The one or more outputs 530 may include the procedure plan 420 but are not limited thereto. For example, the one or more outputs 530 may include related graphics, texts, or graphical user interfaces (GUIs) to display the procedure plan 420, patient anatomy representations, determined and/or enhanced images, etc. configured to be displayed on the display 430 or other output systems 570. Each of these outputs 530 may be used as input preoperative data 522 to determine other outputs 530.

The outputs 530 may be output to any one or more of the output systems 570 via a wireless, electronic, and/or wired connection. The output systems 570 may include the display 430, a mobile device 572, or physical paper, canvas, film or another physical medium 574.

### The Bone Balance Adjustment Algorithm 560

To determine the one or more bone resection parameters 562 and/or implant parameters 564, the bony balancing system 400 may execute the bony balance adjustment algorithm 560. The bony balance adjustment algorithm 560 may be one algorithm and/or may include multiple algorithms that perform similar calculations (e.g., one algorithm for each type of bone cut, soft tissue envelope, etc.) and/or that perform related functions (e.g., bone recognition, surface area calculation, and bone parameter determination). For convenience of description, the bony balance adjustment algorithm 560 will be described as one algorithm, though it will be understood that the bony balance adjustment algorithm 560 may include a lateral laxity algorithm, such as lateral laxity algorithm 576. Although the bony balancing system 400 is described as executing the bony balance adjustment algorithm 560, aspects disclosed herein are not limited to a device or system that executes the bony balance adjustment algorithm 560.

As previously described, the bony balance adjustment algorithm 560 may be configured to determine the one or more bone resection parameters 562 and/or one or more implant parameters 564 for a joint. The bone resection parameters 562 may include a laxity parameter, a bone cut adjustment, a bone cut depth, a bone cut angle or slope, or other adjustments a surgeon may make during a procedure where bone resections are made. The implant parameters 564 may include one or more dimensions of an implant configured to balance the joint and/or a gap of the joint, such as an implant thickness. The bony balance adjustment algorithm 560 may predict a change in laxity of the soft tissue envelope, and adjust (e.g., reduce) the bone resection parameters 562, implant parameters 564, and/or other parameters of the procedure plan 420.

The determination may be based on a learned relationship and/or a linear equation. As an example, the bony balance adjustment algorithm 560 may execute a simple y = mx+b formatted equation, where "x" is the surface area in mm²; "y" is the change in a bone resection depth in millimeters or mm, a change in bone resection angle or slope in degrees, or is the change in an implant thickness in mm; m is a multiplier (e.g., input by the practitioner, learned, and/or refined based on results); and b is a constant. For example, in the context of a knee joint, "b" may be 0 and/or adjusted based on preoperative data 520 (e.g., patient data 524, procedure plan 420), and "m" may be in a range of 0.004 - 0.006 (e.g., 0.005, 0.0055, or 0.0056), but aspects disclosed herein are not limited. For example, "m" and "b" may be different values for different joints and/or adjusted based on patient data. "M" and "b" may also be further adjusted based on learned relationships (e.g., gender) or other preoperative data 520 (e.g., cartilage condition or disease progression), predictions (e.g., cartilage loss or predicted disease progression), intraoperative data, etc. A sign of "m" may depend on a bone resection symmetry and/or a type of parameter being adjusted. For example, where "y" indicates a bone resection depth, "m" may be negative to indicate a decrease in bone resection with respect to that of the initial procedure plan 420. Where "y" indicates an implant thickness, "m" may be positive to indicate an increase in implant thickness with respect to that of the initial procedure plan 420. Where "y" indicates a bone resection angle or slope, the sign of "m" may depend on whether needed resection at one side of the joint is greater than or less than needed resection at an opposite of the joint, as will be described in more detail hereinafter.

For example, the bony balance adjustment algorithm 560 may determine that the removal of bone having a relevant surface area of 90 mm² will result in 0.5 mm of soft tissue laxity based on a linear relationship of 0.5 mm per every 90 mm² and/or 0.1 mm per every 18 mm² (e.g., 180 mm² will result in 1.0 mm of soft tissue laxity, 270 mm² will result in 1.5 mm of soft tissue laxity, 18 mm² will result in 0.1 mm of soft tissue laxity, etc.). In some examples and/or for certain increments, the linear relationship may be simplified and/or rounded up to 0.5 mm per every 100 mm² and/or 0.1 mm per every 20 mm².

The bony balance adjustment algorithm 560 may be configured to determine a linear equation and/or refine the linear equation based on outcomes 450. For example, the bony balance adjustment algorithm 560 may multiply 0.0055 or 0.0056 by the surface area to determine a predicted laxity and/or change in bone resection parameter 562 and/or change in implant parameter 564, but aspects disclosed herein are not limited to this multiplier, which may be adjusted and refined. The bony balance adjustment algorithm 560 may also store an index of surface areas and adjustments (e.g., in a table or database), which may be updated and/or refined based on the outcomes 450.

Certain dimensions of the resected bone, such as width, may not be important as the depth for soft tissue laxity because the soft tissue laxity may correlate with a length gained by the soft tissue not having to travel around the resected bone. For convenience of description, a distance traveled by a hiker hiking over a mountain may primarily depend on two dimensions: (1) a height of the mountain, and (2) a dimension in a forward direction traveled by the hiker. However, a left-right width of the mountain may not be relevant. Similarly, here, only two dimensions may be relevant to the "distance" covered by the soft tissue, while a width may not be relevant. For example, the ligament 206 in FIG. 2 may be stretched primarily in the X and Z directions, but not in a direction into and out of the page. The bony balance adjustment algorithm 560 may therefore not need to approximate all dimensions or a volume of identified bone to be resected and may make a determination primarily based on two dimensions or a determined area. The predetermined view may therefore be a two-dimensional image facing (in a direction perpendicular to) the relevant dimensions to depict an extension of the soft tissue and bone to be resected in the relevant dimensions. In the context of a posterior bone to be resected in a knee joint, this predetermined view may be the sagittal view.

The bony balance adjustment algorithm 560 may also consider a position of the bone planned to be resected to predict the resulting laxity and/or direction of laxity in the soft tissue and/or other bone resection parameters 562. For example, if bone is removed from a posterolateral femur or a posterolateral tibia, then a soft tissue laxity may be lateral. If the bone is resected from a posteromedial femur or a posteromedial tibia, then the soft tissue laxity may be medial. If there are symmetric posterolateral and posteromedial femoral or tibial bone portions to be resected, then the soft tissue laxity may be global. If there are asymmetric posterolateral and posteromedial femoral or tibial bone portions to be removed, then the soft tissue laxity may be asymmetric.

The bone resection parameters 562 may include angular adjustments and/or slopes determined based on the determined surface area of the bone to be resected and/or the determined laxity. For example, if the bony balance adjustment algorithm 560 determines that a portion of bone to be resected of a joint is medial, the bony balance adjustment algorithm 560 may determine that removal of the medial bone portion will create laxity of a medial gap of the joint in flexion and extension. Because some may disagree with the extent of laxity created in flexion in certain joints, in some examples, the bony balance adjustment algorithm 560 may be adjusted and/or consider a practitioner's preference and may determine that removal of the lateral bone portion will create laxity of the lateral gap of the joint primarily in extension, but create less laxity (or none at all) in flexion. For example, the bony balance adjustment algorithm 560 may change a type of bone to which less resection is recommended (e.g., tibia to femur).

In the context of a knee joint, the bony balance adjustment algorithm 560 may determine an alteration of a tibial bone cut (and/or a femoral bone cut, depending on a practitioner's preference) in the procedure plan 420 in anticipation of the determined laxity of the medial gap based on the determined surface area of the medial bone portion. An angular adjustment may correspond to the predicted laxity. For example, if the bony balance adjustment algorithm 560 determines that the surface area of the medial bone portion to be resected is 90 mm², the bony balancing system may determine a bone resection parameter 562 of 0.5° of tibial valgus (or 0.5°of tibial valgus per 0.5 mm of predicted laxity, or 0.1°of tibial valgus per 0.1 mm of predicted laxity) based on a linear relationship of 0.5° per every 90 mm² and/or .1° per every 20 mm² (e.g., 180 mm² will result in 1.0° of tibial valgus, 270 mm² will result in 1.5° of tibial valgus, 20 mm² will result in 0.1° of tibial valgus, etc.). For example, the bony balancing system 400 may multiply 0.0055 or 0.0056 by the surface area to determine a predicted angle or slope (e.g., tibial valgus), but aspects disclosed herein are not limited to this multiplier, which may be adjusted and refined. The bony balance adjustment algorithm 560 may also store an index of surface areas and adjustments, which may be updated and/or refined based on the outcomes 450.

Similarly, if the bony balance adjustment algorithm 560 determines that a portion of bone to be resected of a joint is lateral, the bony balance adjustment algorithm 560 may determine that removal of the lateral portion of bone to be resected will create laxity of a lateral gap of the joint in flexion and extension. Because some may disagree with the extent of laxity created in flexion, in some examples, the bony balance adjustment algorithm 560 may be adjusted and/or consider a practitioner's preference and may determine that removal of the lateral portion of bone will create laxity of the lateral gap of the joint primarily in extension, but create less laxity (or none at all) in flexion.

In the context of a knee joint, the bony balance adjustment algorithm 560 may determine an alteration of a tibial bone cut in the procedure plan 420 in anticipation of the determined laxity of the lateral gap based on the determined surface area of the lateral portion of bone to be resected. An angular adjustment may correspond to the predicted laxity. For example, if the bony balance adjustment algorithm 560 determines that the surface area of the lateral portion of bone to be resected is 90 mm², the bony balancing system may determine a bone resection parameter 562 of 0.5° of tibial varus (or 0.5°of tibial varus per 0.5 mm of predicted laxity) based on a linear relationship of 0.5° per every 90 mm² and/or 0.1° per every 20 mm² (e.g., 180 mm²will result in 1.0° of tibial varus, 270 mm² will result in 1.5° of tibial varus, 20 mm² will result in 0.1° of tibial varus). For example, the bony balance adjustment algorithm 560 may multiply 0.0055 or 0.0056 by the surface area to determine a predicted angle or slope (e.g., tibial varus), but aspects disclosed herein are not limited to this multiplier, which may be adjusted and refined. The bony balance adjustment algorithm 560 may also store an index of surface areas and adjustments, which may be updated and/or refined based on the outcomes 450.

If the bony balance adjustment algorithm 560 identifies symmetric portions of bone to be resected medially and laterally of a joint, then the bony balance adjustment algorithm 560 may determine that removal of these symmetric portions of bone will create global laxity in extension and/or flexion of the joint. In the context of a knee joint, the bony balance adjustment algorithm 560 may determine an alteration of a tibial bone in anticipation of the determined laxity based on the determined surface area of the medial and lateral bone portions. For example, if the bony balance adjustment algorithm 560 determines that the surface area of each of the medial and lateral bone portions are 90 mm², the bony balance adjustment algorithm 560 may determine a tibial resection of the procedure plan 420 to be 0.5 mm less tibial resection based on a linear relationship of 0.5 mm less tibial resection per every 90 mm² and/or 0.1 mm less tibial resection per every 20 mm² (e.g., 180 mm² will result in 1.0 mm less tibial resection, 270 mm² will result in 1.5 mm less of tibial resection, 20 mm² will result in 0.1 mm less of tibial resection, etc.). Alternatively, bony balance adjustment algorithm 560 may determine a liner used in the procedure plan 420 should have a thickness of 0.5 mm more and/or an increased thickness based on the linear relationship as an implant parameter 564. One of ordinary skill in the art will appreciate that liner thicknesses are typically adjusted on the order of 1 mm increments, though this is only exemplary.

If the bony balancing system 400 identifies medial and lateral bone portions to be resected of different size, then the bony balance adjustment algorithm 560 may determine a greater laxity and/or adjustment to the procedure plan 420 (e.g., tibial bone cut) on a side having the larger bone portion. For example, the bony balancing system 400 may determine a difference in a surface area of a medial bone portion and a surface area of a lateral bone portion. Where the medial bone portion is larger than the lateral bone portion and the difference in surface area is 90 mm², the bony balance adjustment algorithm 560 may determine that the procedure plan 420 should include a bone resection parameter 562 of 0.5° of tibial valgus based on a linear relationship of 0.5° per every 90 mm² and/or 0.1° per every 20 mm² (e.g., 180 mm² will result in 1.0° of tibial valgus, 270 mm² will result in 1.5° of tibial valgus, 20 mm² will result in 0.1° of tibial valgus, etc.). Similarly, where the lateral bone portion is larger than the medial bone portion and the difference in surface area is 90 mm², the bony balance adjustment algorithm 560 may determine that the procedure plan 420 should include a bone resection parameter 562 of 0.5° of tibial varus based on a linear relationship of 0.5° per every 90 mm² and/or 0.1° per every 20 mm² (e.g., 180 mm² will result in 1.0° of tibial varus, 270 mm² will result in 1.5° of tibial varus, 20 mm² will result in 0.1° of tibial valgus etc.) etc.

FIG. 7 illustrates an exemplary graphical user interface of the bony balancing system 400 displayed on the display 430. Referring to FIGS. 4-6, a practitioner (e.g., surgeon) may use a computer or other system of the bony balancing system 400 to analyze one or more images of a patient's anatomy 702 (e.g., knee joint) and/or an initially determined procedure plan 420. The one or more images of the patient's anatomy 702 and/or the procedure plan 420 may be included in a case file, patient folder, etc. that is selectable by the practitioner. The one or more images of the patient's anatomy 702 may have been acquired using an image acquisition device 410 and/or with the patient in various poses. The bony balancing system 400 may analyze the one or more images of the patient's anatomy 702 to detect and/or scan to determine a lateral ligament laxity. If the lateral ligament laxity is determined, the bony balancing system 400 may provide a notification or alert 704 (e.g., pop-up or dialogue box) that the lateral ligament laxity was detected. In some examples, the bony balancing system 400 may determine that the planned bone cuts and/or implant will affect soft tissue laxity (e.g., lateral ligament laxity). In such an example, the notification or alert 704 may include text 706 (e.g., "Make adjustments to procedure plan?") offering to make adjustments to the procedure plan 420 based on the detected lateral ligament laxity and present one or more selectable user inputs 708 (e.g., "yes" or "no" buttons) that the practitioner may select. An example of an adjustment to the procedure plan 420 may include whether a target post-operative lateral ligament laxity be substantially the same as the patient's pre-operative lateral ligament laxity, or if the practitioner would prefer to specify a different target post-operative lateral ligament laxity. If the practitioner commands and/or indicates, using the selectable user inputs 708, adjustments to be made, the bony balancing system 400 may execute the bony balance adjustment algorithm 560 to determine the adjustments to the procedure plan 420 and/or to determine a new procedure plan 420.

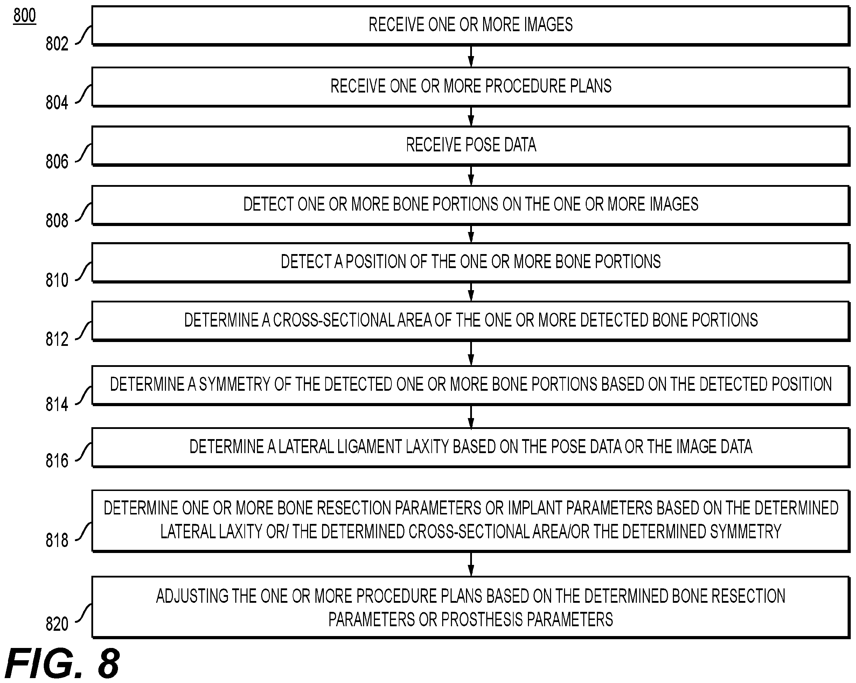

FIG. 8 illustrates an exemplary preoperative method 800 for adjusting a preoperative plan or procedure plan based on a lateral ligament laxity. Referring to FIG. 8, the method 800 may be implemented by the bony balancing system 400 (e.g., using bony balance adjustment algorithm 560) and/or the practitioner. The method 800 may include a step 802 of receiving one or more images of a patient's anatomy, such as one or more CT scans or X-rays. Step 802 may include receiving the one or more images into electronic storage from, for example, an image acquisition device or other system. Step 802 may also include receiving intraoperative image data.

The method 800 may include a step 804 of receiving one or more procedure plans including one or more bone cuts. The procedure plan may also include an implant configured to be received in a bone at or proximate to the one or more bone cuts. Step 804 may include receiving the one or more procedure plans from a practitioner via manual entry, from a remote system that determined the one or more procedure plans, from a memory, and/or may include determining the one or more procedure plans using, for example, the preoperative data 520 described with reference to FIGs 5-6.

The method 800 may include a step 806 of receiving pose data for use by the lateral laxity algorithm and bony balance adjustment algorithm. Pose data may include four total values: (1) a medial extension (e.g., stress test) value, (2) a lateral extension value, (3) medial flexion at the asymptote (e.g., endpoint) of stress value, and (4) lateral side at rest (e.g., in flexion) value. The medial extension value (1) and the lateral extension value (2) may be determined by mechanical stress testing (e.g., valgus and varus stress testing) of the medial and lateral joints. With respect to values (3) and (4), one of ordinary skill in the art will appreciate that varus and valgus stress testing may not be possible due to internal and external rotation of a patient's hip. Value (3) may be determined by identification of the asymptote and/or endpoint by applying either manual or digital distraction force. Value (4) may be identified with a manual distraction force or a digital tensor, or with no distraction, i.e., resting state.

The method 800 may include a step 808 of detecting one or more bone portions on the one or more received images (e.g., bone portions to be resected). Step 808 may include using image processing techniques to identify bone portions and/or machine learning systems trained to detect bone portions (e.g., autonomous segmentation and/or calculation of osteophyte size and location). Alternatively or in addition thereto, step 808 may include receiving input from a practitioner (e.g., via a computer) and/or an annotation indicating one or more bone portions on the one or more received images. Step 808 may include detecting bone portions and identifying whether each detected bone portion is accessible and/or inaccessible for removal before bone cuts. Step 808 may include determining whether each detected bone portion would be inaccessible even after bone cuts. If, in step 808, it is determined that a detected bone portion would be inaccessible even after bone cuts (or highly difficult to access), then the procedure plan and/or predicted soft tissue may be adjusted to account for not removing such bone portion (such that there may be less of a change to soft tissue laxity).

The method 800 may include a step 810 of detecting a position or location of the one or more detected bone portions. Step 810 may include detecting a side or compartment (e.g., medial or lateral) of the one or more detected bone portions relative to a bone, and/or may include determining a coordinate or other positional parameter of the one or more detected bone portions relative to the bone and/or each other where more than one bone portion is detected. Step 810 may include detecting the position using image recognition and/or processing techniques and/or analyzing a plurality of images of the same joint. Alternatively or in addition thereto, step 810 may include receiving input from a practitioner (e.g., via a computer) and/or an annotation indicating the position of the one or more bone portions on the one or more received images.

The method 800 may include a step 812 of determining a cross-sectional or surface area of the one or more detected bone portions. Step 812 may include determining the surface area using image recognition and/or processing techniques and/or analyzing a plurality of images of the same joint. For example, step 812 may include determining or detecting a boundary of the bone portion on an image and calculating an area within the boundary. Alternatively or in addition thereto, step 812 may include receiving input from a practitioner (e.g., via a computer) and/or an annotation indicating the surface area of the one or more bone portions on the one or more received images. The determined cross-sectional area may be output on a display or other output system.

In some examples, step 812 may include identifying an image among a plurality of received images that best reflects the surface area of a portion of bone to be removed. For example, step 812 may include identifying one or more images, among a plurality of received images, which show the patient's anatomy (e.g., joint) across a first predetermined dimension and a second predetermined dimension over which the soft tissue may extend. In the context of a knee joint, the one or more identified images may be sagittal views of the knee joint. Step 812 may further include identifying an image, among the identified images reflecting the first and second predetermined dimensions (e.g., sagittal view), that shows the greatest extent of the bone portion in one of the first and second predetermined dimensions. In some examples, step 812 may include determining a surface area of the detected bone portion based on a plurality of images of a same view (e.g., sagittal view) that include the bone portion, and identifying a maximum surface area calculation, a mean surface area calculation, and/or a median surface area calculation. In some examples, step 812 may include determining a surface area of the detected bone portion based on a plurality of images and/or determining a volume of the detected bone portion, but the algorithm may be simplified by considering surface area in the two most important dimensions, and disregarding a dimension of the bone portion in a dimension that does not affect soft tissue laxity. In other examples, step 812 may include determining a height or depth of the bone portion in one of the predetermined dimensions in place of or in addition to the surface area of the detected bone portion in two dimensions where, for example, the surface area cannot be calculated (e.g., due to poor image quality). In such a situation, the bony balance adjustment algorithm 560 may be adjusted to account for use of only a single dimension to approximate the effect on soft tissue laxity.

The method 800 may include a step 814 of determining a symmetry of the detected bone portions based on the detected positions and/or determined cross-sectional areas. For example, if, in step 806, more than one bone portion is detected, step 814 may include determining that the bone portions are symmetric based on their detected positions and sizes and/or asymmetric based on their detected positions and sizes. Step 814 may include determining that two bone portions are symmetric if they are of a same size or similar size (e.g., a difference in the determined cross-sectional areas is below a predetermined threshold) and are positioned on opposite sides of a joint. Step 814 may include determining that two bone portions are asymmetric if they are of a different size (e.g., a difference in the determined cross-sectional areas is above a predetermined threshold) and/or they occur on a same side of the joint (and/or they occur on sides of the joint that are not opposite). Asymmetry at step 814 may also be determined based on the detected bone portions being different distances or positions away from a predetermined axis. Step 814 may include using image recognition and/or processing techniques and/or analyzing a plurality of images of the same joint. Alternatively or in addition thereto, step 814 may include receiving input from a practitioner (e.g., via a computer) and/or an annotation indicating the symmetry of bone portions on the one or more received images.

The method 800 may include a step 816 of determining a lateral ligament laxity based on the pose data. Step 816 may include using the pose data with the lateral laxity algorithm. In some aspects, the step 816 may be further determined with intraoperative image data using image recognition and/or processing techniques and/or analyzing a plurality of images of the same joint. Alternatively or in addition thereto, step 816 may include receiving input from a practitioner (e.g., via a computer). As described above, the lateral ligament laxity may quantify the amount of lateral laxity in flexion.

The method 800 may include a step 818 of determining one or more bone resection parameters or implant parameters based on the determined lateral ligament laxity and the bony balancing algorithm. Step 818 of determining the one or more bone resection parameters and/or implant parameters may include determining a change in a bone resection parameter and/or implant parameter included in the initially received procedure plan. The determinations made at step 818 may be output on a display or other output system, for example, as a text of instructions and/or list of steps, as a chart, visually on a virtual bone model, etc.

Step 818 may include executing the bony balance adjustment algorithm 560, which may implement one or more linear relationships, as previously described with respect to FIG. 5. The linear relationship may be input and/or refined by the practitioner. Alternatively or in addition thereto, the linear relationship may be learned or refined through assessing multiple patients. In some examples, the linear relationship may be stored and/or implemented using an index database using piecemeal relationships of known surface area amounts, known symmetry, and corresponding changes to the one or more bone resection parameters or implant parameters. In some examples, the linear relationship may include an equation having a multiplier or slope that is multiplied by the surface area to result in the one or more bone resection parameters or implant parameters.

The method 800 may include a step 820 of adjusting the one or more procedure plans based on the determined bone resection parameters and/or implant parameters from step 814. Step 816 may be performed by the bony balance adjustment algorithm 560. Alternatively or in addition thereto, the practitioner may manually adjust the procedure plan based on the determined bone resection parameters and/or implant parameters from step 818. In some aspects, adjustments may be required if asymmetric osteophytes exceed a size difference of 90 mm² or if both medial and lateral osteophytes are larger than 90 mm². To correct global laxity in these cases, it may be necessary to reduce tibial resection or to use a thicker liner. Additionally, tibial cuts (varus or valgus) may need adjustment to address osteophyte size asymmetry.

After steps 818 and 820, the procedure plan may be updated with the adjustments so that the surgeon may perform the procedure plan during surgery. The entire method 800 may be performed before the procedure and before any bone cuts are made. Alternatively or in addition thereto, steps 818 and/or 820 may be repeated based on new intraoperative information received regarding lateral laxity (e.g., identified during surgery but not picked up by the received images). The determinations made during method 800 may be output to a display, for example as a virtual model, a list of steps, as a finalized procedure plan, etc. In some examples, the determinations and/or finalized procedure plan may be output as a set of programmed instructions configured for execution by a robotic platform 440 (e.g., CNC instructions) that may assist with bone cuts or other steps in a medical procedure.

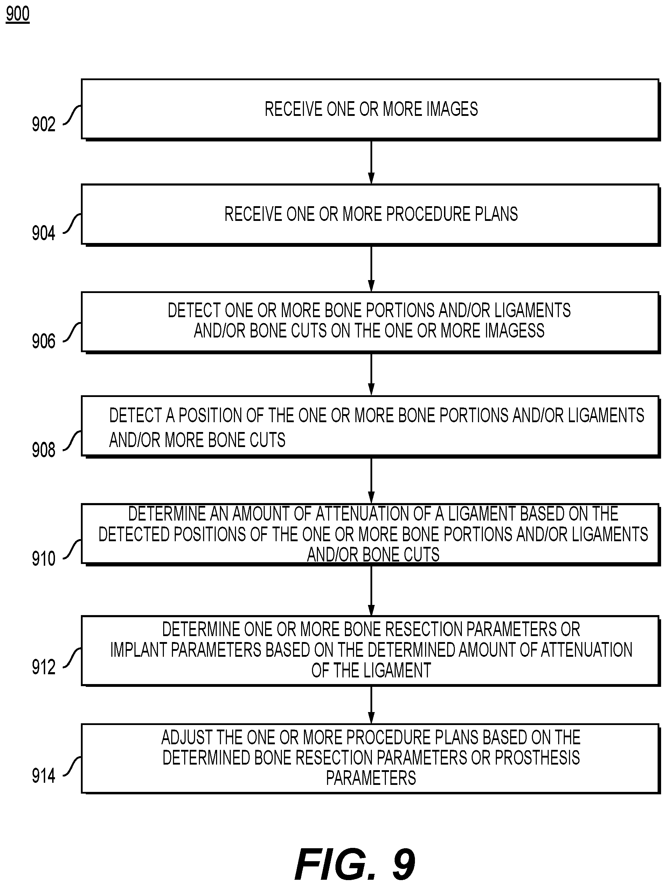

FIG. 9 illustrates an exemplary preoperative method 900 for adjusting a preoperative plan or procedure plan based on a ligament attenuation. Referring to FIG. 9, the method 900 may be implemented by the bony balancing system 400 (e.g., using bony balance adjustment algorithm 560) and/or the practitioner. The method 900 may include a step 902 of receiving one or more images of a patient's anatomy, such as one or more CT scans or X-rays. Step 902 may include receiving the one or more images into electronic storage from, for example, an image acquisition device or other system. Step 902 may also include receiving intraoperative image data.

The method 900 may include a step 904 of receiving one or more procedure plans including one or more bone cuts. The procedure plan may also include an implant configured to be received in a bone at or proximate to the one or more bone cuts. Step 904 may include receiving the one or more procedure plans from a practitioner via manual entry, from a remote system that determined the one or more procedure plans, from a memory, and/or may include determining the one or more procedure plans using, for example, the preoperative data 520 described with reference to FIGs 5-6.

The method 900 may include a step 906 of detecting one or more bone portions and/or one or more ligaments on the one or more received images (e.g., bone portions to be resected). Step 906 may include using image processing techniques to identify bone portions and/or ligaments and/or bone cuts, and/or machine learning systems trained to detect bone portions and/or ligaments (e.g., autonomous segmentation and/or calculation of osteophyte size and location). Alternatively or in addition thereto, step 906 may include receiving input from a practitioner (e.g., via a computer) and/or an annotation indicating one or more bone portions on the one or more received images. Step 906 may include detecting bone portions and/or ligaments and identifying whether each detected bone portion and/or ligament is accessible and/or inaccessible for removal before bone cuts. Step 906 may include determining whether each detected bone portion and/or ligament would be inaccessible even after bone cuts. If, in step 906, it is determined that a detected bone portion and/or ligament would be inaccessible even after bone cuts (or highly difficult to access), then the procedure plan and/or predicted soft tissue may be adjusted to account for not removing such bone portion (such that there may be less of a change to soft tissue laxity).

The method 900 may include a step 908 of detecting a position or location of the one or more detected bone portions and/or the one or more detected ligaments and/or the one or more detected bone cuts. Step 908 may include detecting a side or compartment (e.g., medial or lateral) of the one or more detected bone portions relative to a bone, and/or may include determining a coordinate or other positional parameter of the one or more detected bone portions relative to the bone and/or each other where more than one bone portion is detected. Step 908 may include detecting the position using image recognition and/or processing techniques and/or analyzing a plurality of images of the same joint. Alternatively or in addition thereto, step 908 may include receiving input from a practitioner (e.g., via a computer) and/or an annotation indicating the position of the one or more bone portions and/or one or more ligaments and/or the one or more bone cuts on the one or more received images. Step 908 may include determining the lengths of one or more ligaments and/or an amount of subluxation of one or more bone portions and/or one or more ligaments.

The method 900 may include a step 910 of determining an amount of attenuation of a ligament or joint based on the detected positions and/or measurements of the one or more bone portions and/or ligaments, and/or one or more bone cuts or bone resections. Step 910 may include determining an amount of attenuation by applying attenuation algorithm 577 to the measured/positioned one or more bone portions and/or one or more ligaments and/or one or more bone cuts. For example, step 910 may include determining or detecting an amount of attenuation of a joint caused by subluxation, similarly to the description of FIGs. 10A-10D. Alternatively or in addition thereto, step 910 may include receiving input from a practitioner (e.g., via a computer) and/or an annotation indicating the length of the one or more bone portions and/or the one or more ligaments and/or one or more bone cuts on the one or more received images. The determined lengths can be output on a display or other output system.

In some examples, step 910 may include identifying an image among a plurality of received images that best reflects the surface area of a portion of bone to be removed. For example, step 910 may include identifying one or more images, among a plurality of received images, which show the patient's anatomy (e.g., joint) across a first predetermined dimension and a second predetermined dimension over which the soft tissue may extend. In the context of a knee joint, the one or more identified images may be sagittal views of the knee joint. Step 910 may further include identifying an image, among the identified images reflecting the first and second predetermined dimensions (e.g., sagittal view), that shows the greatest extent of the bone portion in one of the first and second predetermined dimensions. In some examples, step 910 may include determining a surface area of the detected bone portion based on a plurality of images of a same view (e.g., sagittal view) that include the bone portion, and identifying a maximum surface area calculation, a mean surface area calculation, and/or a median surface area calculation. In some examples, step 910 may include determining a surface area of the detected bone portion based on a plurality of images and/or determining a volume of the detected bone portion, but the algorithm may be simplified by considering surface area in the two most important dimensions, and disregarding a dimension of the bone portion in a dimension that does not affect soft tissue laxity. In other examples, step 910 may include determining a height or depth of the bone portion in one of the predetermined dimensions in place of or in addition to the surface area of the detected bone portion in two dimensions where, for example, the surface area cannot be calculated (e.g., due to poor image quality). In such a situation, the bony balance adjustment algorithm 560 may be adjusted to account for use of a single dimension to approximate the effect on soft tissue laxity.

The method 900 may include a step 912 of determining one or more bone resection parameters or implant parameters based on the determined attenuation of the ligament and the bony balancing algorithm. Step 912 of determining the one or more bone resection parameters and/or implant parameters may include determining a change in a bone resection parameter and/or implant parameter included in the initially received procedure plan. The determinations made at step 912 may be output on a display or other output system, for example, as a text of instructions and/or list of steps, as a chart, visually on a virtual bone model, etc.

Step 912 may include executing the bony balance adjustment algorithm 560, which may implement one or more linear relationships, as previously described with respect to FIG. 5. The linear relationship may be input and/or refined by the practitioner. Alternatively or in addition thereto, the linear relationship may be learned or refined through assessing multiple patients. In some examples, the linear relationship may be stored and/or implemented using an index database using piecemeal relationships of known surface area amounts, known symmetry, and corresponding changes to the one or more bone resection parameters or implant parameters. In some examples, the linear relationship may include an equation having a multiplier or slope that is multiplied by the surface area to result in the one or more bone resection parameters or implant parameters.

The method 900 may include a step 914 of adjusting the one or more procedure plans based on the determined bone resection parameters and/or implant parameters from step 912. Step 914 may be performed by the bony balance adjustment algorithm 560. Alternatively or in addition thereto, the practitioner may manually adjust the procedure plan based on the determined bone resection parameters and/or implant parameters from step 912. In some aspects, adjustments may be required if asymmetric osteophytes exceed a size difference of 90 mm² or if both medial and lateral osteophytes are larger than 90 mm². To correct global laxity in these cases, it may be necessary to reduce tibial resection or to use a thicker liner. Additionally, tibial cuts (varus or valgus) may need adjustment to address osteophyte size asymmetry.

After steps 912 and 914, the procedure plan may be updated with the adjustments so that the surgeon may perform the procedure plan during surgery. The entire method 900 may be performed before the procedure and before any bone cuts are made. Alternatively, or in addition thereto, steps 912 and/or 914 may be repeated based on new intraoperative information received regarding ligament attenuation (e.g., identified during surgery but not picked up by the received images). The determinations made during method 900 may be output to a display, for example as a virtual model, a list of steps, as a finalized procedure plan, etc. In some examples, the determinations and/or finalized procedure plan may be output as a set of programmed instructions configured for execution by a robotic platform 440 (e.g., CNC instructions) that may assist with bone cuts or other steps in a medical procedure.

Aspects disclosed herein may provide one or more algorithms to determine a lateral ligament laxity, one or more bone resection parameters (including potential osteophytes) and/or implant parameters, allowing a surgeon to automate preoperative and/or intraoperative decision making. The algorithm(s) may help streamline a workflow for a procedure to anticipate the effect of bone resection parameters and/or implant parameters lateral ligament laxity, and to proactively adjust bone cuts accordingly.

Aspects disclosed herein may be adjusted to a practitioner's preferences and/or methods. For example, some practitioners prefer to create rectangular gaps and/or bone resections, while other practitioners prefer to create trapezoidal gaps and/or bone resection by, for example, making a lateral side looser and/or using a greater bone slope at the lateral side. Aspects disclosed herein may be used to adjust an initial procedure plan that was generated according to the practitioner's preferences, and adjustments themselves may be tweaked based on practitioner preference.

Aspects disclosed herein may provide an algorithm that predicts a lateral ligament laxity based on pose data and/or imaging data. Aspects disclosed herein may provide recommendations based on a correlation between lateral laxity and various bone resection parameters and/or implant parameters. Aspects disclosed herein may provide one or more algorithms to determine one or more bone resection parameters and/or implant parameters and predict soft tissue effects (and/or gap or balance effects) after removal of one or more bone portions and/or posterior bone portion release, allowing a surgeon to automate preoperative and/or intraoperative decision making. The algorithm may help streamline a workflow for a procedure to anticipate the effect of removal of one or more bone portions (e.g., posterior bone portion) on soft tissue balancing, and to proactively adjust bone cuts accordingly. Aspects of this disclosure may be applicable to bone resection of osteophytes.

Aspects disclosed herein may provide an algorithm that calculates one or more bone resection parameters and/or one or more implant parameters based on a cross-sectional or surface area of a bone portion. The linear relationship may include adjusting parameters by a predetermined amount per a predetermined amount of cross-sectional area. For example, the linear relationship may include adjusting a bone resection parameter and/or implant parameter by 0.5° and/or 0.5 mm per every 90 mm² of cross-sectional area of the bone portion, or 0.1° and/or 0.1 mm per every 20 mm² of cross-sectional area of the bone portion, but aspects disclosed herein are not limited. For example, the predetermined amount in parameters may be in a range of 0.4° - 0.6° and/or 0.4 mm - 0.6 mm, and the predetermined amount of cross-sectional area may be in a range of 80 mm² or 85 mm² to 100 mm², etc. As another example, the predetermined amount in parameters may be in a range of 0.075° - .125° and/or 0.075 mm -.125 mm, and the predetermined amount of cross-sectional area may be in a range of 15 mm² to 25 mm², etc. The linear relationship may correspond to y=mx+b or y=mx, where "m" may be 0.0055 or 0.0056, or in a range of 0.005 - 0.006 or 0.004-0.006 (for example, in a context of a knee joint). For other joints, "m" and/or the linear relationship may differ based on a learned relationship.

Aspects disclosed herein may be adjusted to a practitioner's preferences and/or methods. For example, some practitioners prefer to create rectangular gaps and/or bone resections, while other practitioners prefer to create trapezoidal gaps and/or bone resection by, for example, making a lateral side looser and/or using a greater bone slope at the lateral side. Aspects disclosed herein may be used to adjust an initial procedure plan that was generated according to the practitioner's preferences, and adjustments themselves may be tweaked based on practitioner preference.

Aspects disclosed herein may assist practitioners in planning bone cuts for patients undergoing a joint replacement surgery to correct a varus or valgus deformity, where the patient's specific lateral ligament laxity may significantly affect balancing the patient's knee and post-operative outcomes. Aspects disclosed herein may consider an extent of deformity in determining modifications to bone resection parameters and/or implant thickness to account for bone portion removal.

Aspects disclosed herein may provide an algorithm that predicts increases in an extension gap and flexion gap (or, alternatively, an extension gap) after removal of one or more posterior bone portions based on a cross-sectional area of the one or more posterior bone portions. Aspects disclosed herein may provide recommendations based on a correlation between bone portion size and/or location and soft tissue affect.

Aspects disclosed herein may simplify a prediction of a soft tissue effect caused by bone portion removal by considering a cross-sectional area at a largest part of the bone portion (e.g., using one slice of the CT scan) and by not requiring more dimensions and/or calculations (e.g., a bone portion volume). Aspects disclosed herein may not require consideration of a width of the bone portion and/or an entire volume of the bone portion.

Aspects disclosed herein may provide recommendations regardless of whether a deformity is fixed and/or flexible and may not require input of whether a deformity is fixed or flexible. Aspects disclosed herein make predictions on an effect on soft tissue laxity that is independent of a rigidity of the deformity. Aspects disclosed herein may provide a linear correlation between a size (e.g., surface area and/or cross-sectional area) of a bone portion removed and a resultant soft tissue laxity.

Aspects disclosed herein may provide recommendations regardless of patient reported outcomes and may not require input of patient reported outcomes. Aspects disclosed herein may correlate outcome with bone portion size based on a correlation between bone portion volume and disease (e.g., severity or progression). Aspects disclosed herein may predict a greater outcome based on removal of a larger bone portion.

Aspects disclosed herein may identify a direction and a magnitude of a deformity and an anticipated result of bone portion removal. Aspects disclosed herein may provide a predictive algorithm and/or provide granular detail to identify a direction and a magnitude of a deformity to account for posterior bone portion removal and subsequent increase in an extension and/or flexion gap. Aspects disclosed herein may provide an algorithm configured to characterize ae scope of deformity both in direction and magnitude.

Aspects disclosed herein may be used to sense or collect preoperative, intraoperative, and/or postoperative information about a patient and/or a procedure.

Aspects disclosed herein contemplate implants or prosthetics and are not limited to the contexts described. For example, implants disclosed herein may be implemented as another implant system for another joint or other part of a musculoskeletal system (e.g., hip, knee, spine, bone, ankle, wrist, fingers, hand, toes, or elbow) and/or as sensors configured to be implanted directly into a patient's tissue, bone, muscle, ligaments, etc. Each of the implants or implant systems may include sensors such as inertial measurement units, strain gauges, accelerometers, ultrasonic or acoustic sensors, etc. configured to measure position, speed, acceleration, orientation, range of motion, and/or sensors configured to measure biometric changes (e.g., color change, pH change, etc.) in synovial fluid, blood glucose, temperature, or other biometrics and/or may include electrodes that detect current information, ultrasonic or infrared sensors that detect other nearby structures, etc. to detect an infection, invasion, nearby tumor, etc. The implants may, for example, be a sensor or other measurement device configured to be drilled into a bone, another implant, or otherwise implanted in the patient's body.

Aspects and systems disclosed herein may make determinations based on images or imaging data (e.g., from CT scans). Aspects disclosed herein may predict soft tissue laxity, a resulting gap, bone resection and/or implant parameters, etc. based on one slice or view of a CT scan and may not require calculating a bone portion volume, etc. Images disclosed herein may display or represent bones, tissues, or other anatomy, and systems and aspects disclosed herein may recognize, identify, classify, and/or determine portions of anatomy such as bones, cartilage, tissue, and bone landmarks, such as each specific vertebra in a spine. Aspects and systems disclosed herein may determine relative positions, orientations, and/or angles between recognize bones, such as a Cobb angle, an angle between a tibia and a femur, and/or other alignment data.

Aspects and systems disclosed herein provide displays having graphical user interfaces configured to graphically display data, determinations, and/or steps, targets, instructions, or other parameters of a procedure, including preoperatively, intraoperatively, and/or postoperatively. Figures, illustrations, animations, and/or videos displayed via user interfaces may be recorded and stored on the memory system.

Aspects and systems disclosed herein may be implemented using machine learning technology. One or more algorithms may be configured to learn or be trained on patterns and/or other relationships across a plurality of patients in combination with preoperative information and outputs, intraoperative information and outputs, and postoperative information and outputs. The learned patterns and/or relationships may refine determinations made by one or more algorithms and/or also refine how the one or more algorithms are executed, configured, designed, or compiled. The refinement and/or updating of the one or more algorithms may further refine displays and/or graphical user interfaces (e.g., bone recognition and/or determinations, targets, recognition and/or display of other conditions and/or bone offsets, etc.).

Aspects disclosed herein may be configured to optimize a "fit" or "tightness" of an implant provided to a patient during a medical procedure based on detections by the one or more algorithms. A fit of the implant may be made tighter by aligning the implant with a shallower bone slope and/or determining a shallower resulting or desired bone slope, by increasing a thickness or other dimensions of the implant, by determining certain types of materials or a type of implants or prosthesis (e.g., a stabilizing implant, a VVC implant, an ADM implant, or an MDM implant). A thickness of the implant may be achieved by increasing (or decrease) a size or shape of the implant. Tightness may be impacted by gaps and/or joint space width, which may be regulated by an insert which may vary depending on a type of implant or due to a motion. Gaps may be impacted by femoral and tibial cuts. Tightness may further be impacted by slope. A range of slope may be based on implant choice as well as surgical approach and patient anatomy. A thickness of the implant may also be achieved by adding or removing an augment or shim. For example, augments or shims may be stackable and removable, and a thickness may be increased by adding one or more augments or shims or adding an augment or shim having a predetermined (e.g., above a certain threshold) thickness. Fit or tightness may also be achieved with certain types of bone cuts, bone preparations, or tissue cuts that reduce a number of cuts made and/or an invasiveness during surgery.

Aspects disclosed herein may be implemented during a robotic medical procedure using a robotic device. Aspects disclosed herein are not limited to specific scores, thresholds, etc. that are described. For example, outputs and/or scores disclosed herein may include other types of scores such as the hip disability and osteoarthritis score or HOOS, KOOS, SF-12, SF-36, Harris Hip Score, etc.

Aspects disclosed herein are not limited to specific types of surgeries and may be applied in the context of osteotomy procedures, computer navigated surgery, neurological surgery, spine surgery, otolaryngology surgery, orthopedic surgery, general surgery, urologic surgery, ophthalmologic surgery, obstetric and gynecologic surgery, plastic surgery, valve replacement surgery, endoscopic surgery, and/or laparoscopic surgery.

Aspects disclosed herein may improve or optimize surgery outcomes, implant designs, and/or preoperative analyses, predictions, or workflows. Aspects disclosed herein may augment the continuum of care to optimize post-operative outcomes for a patient. Aspects disclosed herein may recognize or determine previously unknown relationships, to help optimize care, predict soft tissue laxity, and/or to optimize design of a prosthetic or implant.

## Claims

1. A method of assessing a joint, comprising:
identifying a first bone portion in an image of the joint, wherein the first bone portion is positioned under a soft tissue;
identifying a cross-sectional area of the first bone portion;
identifying a medial ligament in extension value, a lateral ligament in extension value, a medial ligament in flexion value, a lateral side in flexion value associated with the joint;
determining a lateral ligament laxity based on the lateral ligament in extension value;
determining one or more adjustment parameters based on the identified cross-sectional area of the first bone portion and the lateral ligament laxity, wherein determining the one or more adjustment parameters includes applying a linear equation that receives, as input, the identified cross-sectional area and the lateral ligament laxity, wherein the one or more adjustment parameters include:
a predicted change in soft tissue laxity after the identified first bone portion is removed, wherein the soft tissue laxity includes the lateral ligament laxity;
an adjustment to a planned bone resection of one or more bone cuts;
an adjustment to a planned bone resection angle of the one or more bone cuts; and/or
an adjustment to a planned thickness of an implant; and
outputting the one or more determined adjustment parameters to a display.

2. The method of claim 1, wherein identifying the first bone portion and/or identifying the cross-sectional area of the first bone portion includes analyzing the image using one or more image processing techniques.

3. The method of claim 1, wherein identifying the cross-sectional area of the first bone portion includes analyzing a first dimension of the first bone portion and a second dimension of the first bone portion, and disregarding a third dimension of the first bone portion.

4. The method of claim 1, wherein the one or more adjustment parameters include an adjustment to a planned bone resection depth of the one or more bone cuts and an adjustment to a planned bone resection angle of the one or more bone cuts, wherein identifying the cross-sectional area of the first bone portion includes determining that the image of the joint is an image of a set of images showing a greatest extent of the first bone portion in a first dimension.

5. The method of claim 1, further comprising:
identifying a ligament in the image of the joint;
identifying a measurement of the ligament and (i) an amount of subluxation of the first bone portion or (ii) a measurement of a bone cut of the first bone portion; and
identifying an amount of attenuation of the ligament, based on the measurement of the ligament and at least one of (i) the amount of subluxation of the first bone portion or (ii) a measurement of a bone cut of the first bone portion,
wherein the determining the one or more adjustment parameters is further based on the amount of attenuation of the joint, and
wherein the linear equation further receives as input the amount of attenuation of the joint.

6. The method of claim 1, wherein the linear equation includes:
a linear relationship between the identified cross-sectional area and an adjustment to a planned bone resection depth such that, the greater the identified cross-sectional area, the greater the decrease in the planned bone resection depth; and/or
a linear relationship between the identified cross-sectional area and the planned thickness of the implant such that, the greater the identified cross-sectional area, the greater the increase to the planned thickness of the implant.

7. The method of claim 1, further comprising:
identifying a second bone portion in the image of the joint that is positioned under the soft tissue;
identifying a cross-sectional area of the second bone portion;
identifying a position of the first bone portion; and
identifying a position of the second bone portion;
wherein determining the one or more adjustment parameters is further based on the identified cross-sectional area of the second bone portion, the identified position of the first bone portion, and the identified position of the second bone portion.

8. The method of claim 7, further comprising:
determining, based on the identified position of the first bone portion and the identified position of the second bone portion, the first bone portion is provided at a first side of the joint and the second bone portion is provided at a second side of the joint opposite the first side; and
determining a difference in the identified cross-sectional area of the first bone portion and the identified cross-sectional area of the second bone portion, wherein the linear equation includes a linear relationship between the determined difference in the identified cross-sectional areas and the adjustment to the planned bone resection angle such that, the greater the determined difference in the identified cross-sectional areas, the greater the adjustment to the planned bone resection angle.

9. The method of claim 8, further comprising determining whether a difference in the identified cross-sectional areas is greater than or equal to a predetermined difference threshold, wherein:
if the determined difference in the identified cross-sectional area is not greater than or equal to the predetermined difference threshold, determining that the first bone portion and the second bone portion are symmetric; and
if the determined difference in the identified cross-sectional area is greater than the predetermined difference threshold, determining that the first bone portion and the second bone portion are asymmetric.

10. The method of claim 9, wherein:
if the first bone portion and the second bone portion are determined to be symmetric, executing an algorithm including:
determining that a planned bone resection depth should be decreased by a predetermined amount of depth per a predetermined amount of the identified cross-sectional area of the first bone portion and/or the second bone portion, or
determining that the planned thickness of the implant should be increased by the predetermined amount of depth per the predetermined amount of the identified cross-sectional area of the first bone portion and/or the second bone portion; and
if the first bone portion and the second bone portion are determined to be asymmetric, then executing the algorithm includes:
determining, based on the identified cross-sectional area of the first bone portion and the identified cross-sectional area of the second bone portion, whether the first bone portion is larger than the second bone portion;
if the first bone portion is determined to be larger than the second bone portion, determining that the planned bone resection angle should be adjusted in a first direction or orientation by a predetermined amount of bone resection angle per a predetermined amount of difference between the identified cross-sectional area of the first bone portion and the identified cross-sectional area of the second bone portion; and
if the first bone portion is determined not to be larger than the second bone portion, determining that the second bone portion is larger than the first bone portion, and determining that the planned bone resection angle should be adjusted in a second direction or orientation opposite the first direction or orientation by the predetermined amount of bone resection angle per the predetermined amount of difference.

11. The method of claim 10, further comprising:
determining that the first bone portion and the second bone portion are asymmetric;
determining that both the identified cross-sectional area of the first bone portion and the identified cross-sectional area of the second bone portion are greater than a predetermined cross-sectional area;
determining that the difference in the identified cross-sectional areas is greater than a predetermined difference; and
determining that the planned bone resection depth should be decreased by the predetermined amount of depth per the predetermined amount of the identified cross-sectional area of the first bone portion and/or the second bone portion, or
determining that the planned thickness of the implant should be increased by the predetermined amount of depth per the predetermined amount of the identified cross-sectional area of the first bone portion and/or the second bone portion.

12. The method of claim 10, wherein the joint is a knee joint, and:
the first bone portion is a lateral bone portion and the second bone portion is a medial bone portion;
the one or more bone cuts includes a tibial bone cut or a femoral bone cut;
the first direction is a tibial varus or a femoral varus; and
the second direction is a tibial valgus or a femoral valgus.

13. The method of claim 12, wherein:
the predetermined amount of depth is in a range of 0.4 millimeters (mm) to 0.6 mm;
the predetermined amount of the identified cross-sectional area of the first bone portion and/or the second bone portion is in a range of 85 mm² to 100 mm²;
the predetermined amount of bone resection angle is in a range of 0.4° to 0.6°; and
the predetermined amount of difference between the identified cross-sectional area of the first bone portion and the identified cross-sectional area of the second bone portion is in a range of 80 mm² to 100 mm²,
optionally, wherein:
the predetermined amount of depth is in a range of 0.075 mm - 0.125 mm;
the predetermined amount of the identified cross-sectional area of the first bone portion and/or the second bone portion is in a range of 15 mm² to 25 mm²;
the predetermined amount of bone resection angle is in a range of 0.075° - 0.125°; and
the predetermined amount of difference between the identified cross-sectional area of the first bone portion and the identified cross-sectional area of the second bone portion is in a range of 15 mm² to 25 mm².

14. A method of assessing a joint, comprising:
identifying a first bone portion and a ligament in an image of the joint, wherein the first bone portion is positioned under the ligament;
identifying a cross-sectional area of the first bone portion;
identifying a measurement of the ligament and an amount of subluxation of the first bone portion; and
identifying an amount of attenuation of the ligament, based on the measurement of the ligament and the amount of subluxation of the first bone portion,
determining one or more adjustment parameters based on the identified cross-sectional area of the first bone portion and the amount of attenuation of the joint, wherein determining the one or more adjustment parameters includes applying a linear equation that receives, as input, the identified cross-sectional area and the amount of attenuation of the ligament, wherein the one or more adjustment parameters include:
a predicted change in soft tissue laxity after the identified first bone portion is removed;
an adjustment to a planned bone resection of one or more bone cuts;
an adjustment to a planned bone resection angle of the one or more bone cuts; and/or
an adjustment to a planned thickness of an implant; and
outputting the one or more determined adjustment parameters to a display,
optionally, wherein the one or more bone resection parameters include a planned bone resection depth of the one or more bone cuts and/or a planned bone resection angle of the one or more bone cuts.

15. A system configured to assess a joint, comprising:
an image acquisition device configured to acquire at least one image of the joint;
a memory configured to store information, the information including imaging data related to the at least on acquired image and pose parameters, wherein the imaging data includes a cross-sectional area and position of at least one bone portion of the joint positioned under a soft tissue, wherein the pose parameters include a medial ligament in extension value, a lateral ligament in extension value, a medial ligament in flexion value, and a lateral ligament in flexion value;
a controller configured to:
execute a first algorithm to determine a lateral ligament laxity, wherein the first algorithm receives, as input, the at least one image and the lateral ligament in flexion; and
execute a second algorithm to determine, based on the lateral ligament laxity, the at least one acquired image, and/or the stored imaging data, one or more adjustment parameters, wherein the second algorithm applies a linear equation that receives, as input, the lateral ligament laxity and the cross-sectional area of the at least one bone portion, and outputs the one or more adjustment parameters, wherein the one or more adjustment parameters include an adjustment to a bone resection parameter and/or an adjustment to an implant parameter; and
a display configured to display the determined one or more adjustment parameters,
optionally, wherein the image acquisition device is a computed tomography (CT) acquisition device, and the acquired at least one image is a CT scan
optionally, wherein the CT scan shows a view of the joint in a first dimension and a second dimension over which the soft tissue extends, and the cross-sectional area is determined using the dimension of the bone portion in the first dimension and the second dimension.
optionally, wherein the display is configured to display a graphical user interface that includes a notification based on one or more bone portions identified in the acquired image.
